# EUROPÄISCHE PATENTSCHRIFT

(11) **EP 3 559 922 B1**
(45) Veröffentlichungstag und Bekanntmachung des Hinweises auf die Patenterteilung: **17.07.2024**
(21) Anmeldenummer: 17811493.0
(22) Anmeldetag: 23.11.2017
(51) Int. Cl.: A47C 7/70, A47C 7/74, A47L 1/02, A47L 7/00, A47L 9/12, A47L 9/19, A47L 9/28, G08B 21/04, G08B 25/01, A61B 5/11, A61B 5/00, A47L 11/40, A47C 7/72, A61G 5/00, A47C 7/50, A61B 5/0205, G05D 1/00, B01F 27/80, B01F 33/84, A61F 4/00

(54) **SET AUS SITZ- ODER LIEGEMÖBEL UND EINEM AUTONOMEN HAUSHALTSGERÄT**
SET COMPRISING SEATING OR RECLINING FURNITURE AND AUTONOMOUS DOMESTIC APPLIANCE
ENSEMBLE COMPRENANT UN MEUBLE DESTINÉ À S'ASSEOIR ET/OU À S'ALLONGER ET APPAREIL ÉLECTROMÉNAGER AUTONOME

(30) Priorität: 21.12.2016 DE 102016125199; 08.03.2017 DE 102017104846
(43) Veröffentlichungstag der Anmeldung: 30.10.2019
(73) Patentinhaber: Service-Konzepte MM AG, 30539 Hannover (DE)
(72) Erfinder: MÖWISCH, Anja, 30539 Hannover (DE)
(74) Vertreter: Kröncke, Rolf
(86) Internationale Anmeldenummer: PCT/EP2017/080231
(87) Internationale Veröffentlichungsnummer: WO 2018/114209

(56) Entgegenhaltungen:
- EP-A1- 1 566 782
- EP-A1- 2 933 953
- EP-A2- 2 407 074
- WO-A1-2008/047078
- WO-A1-2008/059432
- WO-A1-2009/058839
- WO-A1-2013/130576
- WO-A1-2013/150070
- WO-A2-2008/057618
- CN-A- 106 037 326
- DE-A1- 102011 087 589
- DE-A1- 19 614 916
- US-A- 6 002 994
- US-A1- 2013 100 268
- US-A1- 2014 091 604
- US-A1- 2015 223 705
- US-A1- 2016 058 245
- "COMPUTER-IN-AN-ARMCHAIR", IBM TECHNICAL DISCLOSURE BULLETIN, INTERNATIONAL BUSINESS MACHINES CORP. (THORNWOOD), US, vol. 32, no. 9B, 1 February 1990 (1990-02-01), pages 305 - 307, XP000082361, ISSN: 0018-8689

## Beschreibung

Die Erfindung betrifft ein Set aus einem Sitz- oder Liegemöbel für eine zu betreuende Person und einem autonomen Haushaltsgerät, wobei das Sitz- oder Liegemöbel mit einer schwenkbar, verschiebbar und/oder teleskopierbar an dem Sitz- oder Liegemöbel angeordneten Serviceeinheit ausgestattet ist und die Serviceeinheit einen Monitor und eine Eingabeeinheit hat. Das autonome Haushaltsgerät ist in Form eines Bodenreinigungsroboters oder Rasenmähroboters mit einer Plattform, die Fortbewegungsmittel, Umgebungssensoren zur Detektion von Eigenschaften der Umgebung des autonomen Haushaltsgerätes und eine Steuerungseinheit hat, wobei die Umgebungssensoren mit der Steuerungseinheit verbunden sind und die Steuerungseinheit zur autonomen Fortbewegung des Haushaltsgerätes durch Ansteuerung der Fortbewegungsmittel in Abhängigkeit von den detektierten Umgebungseigenschaften und zum ständigen Abfahren eines Gebietes zur Ausführung seiner eigentlichen Haushaltstätigkeit eingerichtet ist.

Solche autonomen Haushaltsgeräte sind an sich als Saugroboter zur Bodenreinigung in Gebäuden oder Mährobotern zum autonomen Mähen von Rasenflächen hinreichend bekannt. Sie sind durch geeignete Sensoren, wie Tastsensoren, induktive Grenzliniensensoren, Ultraschallsensoren, optische Sensoren oder Ortungssensoren so eingerichtet, dass sie Hindernisse einschließlich von Stufen und Vertiefungen erkenn und umfahren und über eine längere Zeit eine zusammenhängende Fläche mindestens einmal bearbeitet haben.

DE 10 2011 053 990 A1 beschreibt beispielsweise eine elektrische Küchenmaschine, in der Rezepte zum Aufruf abgespeichert sind.

Bei der Patientenversorgung besteht ein Bedarf, um die ärztlich verordnete Eingabe von Medikamenten im Tagesablauf sicherzustellen und zu überwachen. Hierzu ist in DE 20 2006 004 282 U1 ein Arzneimittelspender für die Zuteilung von Medikamenten beschrieben, der einen vom Funktionsschalter ausgelösten Entriegelungsmechanismus zur Medikamentenausgabe hat.

DE 20 2012 000 410 U1 beschreibt ein System für die geriatrische Notfall- und Vitalparameterkommunikation zur Kontrolle der Medikamenteneinnahme eines Patienten, das als tragbares Gerät ausgestaltet ist.

DE 10 2015 100 224 A1 offenbart ein implantierbares Medikamentenapplikationssystem, das operativ in einen Hohlraum des Körpers eingesetzt werden muss.

US 2016/0058245 A1 zeigt ein Gerät, das ausschließlich zur Zubereitung von Energiedrinks mit Nahrungsergänzungsmitteln vorgesehen ist, nicht aber zur Medikamentengabe.

EP 0 753 160 B1 beschreibt ein Verfahren und ein Gerät zur Hinderniserkennung für ein solches autonomes Gerät. Dabei ist mindestens ein Mikrofon vorgesehen, um mit akustischen Methoden Hindernisse zu erkennen.

Zur Überwachung von hilfsbedürftigen Personen sind zudem im Gebäude installierte oder von der zu betreuenden Person am Körper zu tragende mobile Not- und Hilferufsysteme bekannt. Damit soll Menschen, die z. B. aufgrund von Herzkreislaufstörungen, Spastiken, Tremor und Demenz eingeschränkt sind, ein eigenständiges Leben in der eigenen Wohnung ermöglicht werden. Ein solches Notsystem ist beispielsweise in DE 195 17 037 A1 beschrieben.

US 2015/0273697 A1 und US 2005/0216126 A1 offenbaren autonome Serviceroboter, die zur Unterstützung von zu versorgenden Personen speziell ausgestaltet sind und hierzu eine sehr robuste Plattform haben.

US 2005/0154265 A1 beschreibt einen Roboter, der als Schnittstelle zwischen einem Arzt oder einer Pflegekraft und einem Patienten dient, um mit Spracherkennung Wünsche aufzunehmen und weiterzugeben.

US 5,553,609 A beschreibt ein Patientenüberwachungssystem mit Videokameras, Mikrofonen und Lautsprechern, die mit einer Überwachungszentrale verbunden sind.

US 5,802,494 A offenbart ein entsprechendes Patientenmonitorsystem, das zusätzlich spezialisierte Pflegeroboter zur Patientenunterstützung aufweist.

US 2006/0176182 A1 zeigt einen hundeartigen Roboter mit einer Kamera, der zur Überwachung des Gesundheitszustandes und der Sicherheit von Personen speziell ausgestaltet ist.

In US 2016/0166577 A1 sind fliegende Drohnen zur Überwachung beschrieben.

US 2013/0245827 A1 offenbart ein ferngesteuertes Überwachungssystem mit einem fahrbaren Überwachungsgerät, das ein Gebiet innerhalb einer vorgegebenen räumlichen Zone überwacht und eine Alarmmeldung abgibt, wenn z. B. ein Kleinkind diese Zone überschreitet. Im Überwachungsmodus positioniert sich das Gerät im Bereich der vorgegebenen Grenze. DE 10 2011 087 589 A1 beschreibt ein modulares Assistenzsystem für eine Person, das mindesten eine mobile Einheit hat, die wahlweise mit austauschbaren Funktionsmodulen zur Versorgung der Person, mit Messmodulen, mit von der Person fernsteuerbarem Reinigungsmodul usw. ausgerüstet werden kann.

Ein Problem tragbarer Not- und Hilfseinrichtungen, die z. B. wie eine Armbanduhr von der zu betreuenden Person mitgeführt werden müssen, besteht in der Akzeptanz. Die betreuende Person muss für die Sicherstellung der Funktionalität gewährleisten, dass sie die Notrufeinheit auch mitführt. Die Festinstallation von Notrufanlagen in Gebäuden ist aufwendig und bedarf ebenfalls der Akzeptanz.

WO 2008/057618 A2 beschreibt einen ergonomisch ausgebildeten Sessel mit einer Halterung, an der ein Bildschirm eines Computers angeordnet ist.

CN 106 037 326 A offenbart einen zur drahtlosen Kommunikation ausgebildeten Multifunktionssessel mit integrierten Lautsprechern und Mikrophon.

US 2014/091604 A1 offenbart einen Cockpitsitz für ein Flugzeug mit einer beweglich an dem Cockpitsitz angeordneten Steuereinheit in Form eines Tabletcomputers mit berührungsempfindlichem Bildschirm (Touch-Screen-Tablet), der zur Kommunikation mit Steuereinheiten des Flugzeugs ausgebildet ist.

Die Veröffentlichung ""COMPUTER-IN-AN-ARMCHAIR", IBM TECHNICAL DISCLOSURE BULLETIN, INTERNATIONAL BUSINESS MACHINES CORP. (THORNWOOD), US, Bd. 32, Nr. 9B, 1. Februar 1990, Seiten 305-307" beschreibt einen Sessel für eine zu betreuende Person mit integriertem PC.

Ausgehend hiervon ist es daher Aufgabe der vorliegenden Erfindung, eine zuverlässige und möglichst einfache sowie kaum wahrnehmbare Hilfseinrichtung für bedürftige Personen zu schaffen.

Die Aufgabe wird mit dem Set mit den Merkmalen des Anspruchs 1 gelöst. Vorteilhafte Ausführungsformen sind in den Unteransprüchen beschrieben.

Es wird vorgeschlagen, ein Sitz- oder Liegemöbel als Ladestation für ein autonomes Haushaltsgerät zu nutzen.

Dabei wird das gattungsgemäße autonome Haushaltsgerät als Plattform ergänzend zur eigentlichen Haushaltstätigkeit zur Erkennung der Hilfsbedürftigkeit einer Person in Abhängigkeit von Umgebungseigenschaften genutzt, die mit Umgebungssensoren des autonomen Haushaltsgeräts erfassbar sind. Das autonome Haushaltsgerät hat dann eine Kommunikationseinheit, die zum Aufbau einer Kommunikationsverbindung mit einer entfernt angeordneten Serviceeinheit eingerichtet ist. Die Kommunikationseinheit kann zur drahtlosen Funkkommunikation mit einer Funksendeeinheit oder einem Tranceiver eingerichtet sein. Optional hierzu oder ergänzend zur drahtlosen Kommunikation kann die Kommunikationseinheit auch zum drahtgebundenen Datenaustausch mit der Serviceeinheit eingerichtet sein, z. B. indem das autonome Haushaltsgerät in Kontakt mit einer Dockingstation tritt, um ein Auslesen der in dem autonomen Haushaltsgerät zwischengespeicherten Daten zu ermöglichen. Die Dockingstation kann bevorzugt eine Ladestation für das autonome Haushaltsgerät sein. Damit kann ein abhör- und manipulationsgesicherter Datenaustausch sichergestellt werden. Bei der drahtlosen Kommunikation kann dies durch verschlüsselte und/oder zertifizierte Datenübertragung gewährleistet werden.

Das Haushaltsgerät ist ein Bodenreinigungsroboter oder ein Mähroboter, der eine Bodenreinigungs- oder Rasenmäheinheit hat. Ein solcher Bodenreinigungsroboter kann beispielsweise ein Staubsauger, ein Flächenwischroboter oder eine Kombination hiervon sein. Ein solcher Flächenwischroboter kann beispielsweise ein Bodenwischroboter oder sogar ein Fensterreinigungsroboter sein.

Das autonome Haushaltsgerät fährt, wie bei Saugrobotern oder Mährobotern üblich, ständig das zugewiesene Gebiet ab und überwacht dabei ergänzend zur eigentlichen Haushaltstätigkeit noch mithilfe der Umgebungssensoren die Hilfsbedürftigkeit einer Person.

Die Erkennung der Hilfsbedürftigkeit einer Person kann mit einer hierzu eingerichteten Personenhilfseinheit erfolgen, die z. B. durch modularen Anbau an das autonome Hilfsgerät in dieses integriert und zur drahtlosen Übermittlung einer Hilfeanforderung über die Kommunikationseinheit im Falle einer erkannten Hilfebedürftigkeit eingerichtet ist. Alternativ kann die Erkennung der Hilfsbedürftigkeit auch durch die als Personenhilfseinheit ausgebildete entfernte Serviceeinheit anhand der von dem autonomen Haushaltsgerät drahtlos empfangenen Umgebungssensordaten erfolgen. Das autonome Haushaltsgerät wirkt somit durch Detektion von Umgebungseigenschaften bei der letztendlich durch die entfernte Serviceeinheit durchgeführte Erkennung der Hilfsbedürftigkeit mit.

Mithilfe der autonom bewegbaren Plattform kann sich das autonome Haushaltsgerät mit seinen Umgebungssensoren in einem Raumbereich selbstständig bewegen und auf Anforderung z. B. bei einem erkannten Hilferuf zu der hilfsbedürftigen Person fahren und die Hilfsbedürftigkeit verifizieren und melden. Der Raumbereich, d. h. das Gebiet, in dem das autonome Haushaltsgerät seine eigentliche Haushaltstätigkeit selbstfahrend ausführt, ist durch die Hindernis- oder Grenzerkennung festgelegt. Er wird durch die Positionierung des Haushaltsgebietes zugewiesen und kann durch Grenzmarkierungen eingegrenzt werden.

Die Personenhilfseinheit fällt dabei nicht auf, so dass sich die zu betreuende Person nicht völlig überwacht und beobachtet fühlt und jeder Außenstehende erkennt, dass aufgrund von gesundheitlichen Einschränkungen Pflegehilfsmittel notwendig sind. Bereits vor Beginn der Pflegebedürftigkeit genutzte Haushaltsgeräte können als Plattform weitergenutzt werden und werden hierdurch akzeptiert. Die Kommunikationseinheit und eine Einheit zur Detektion von Umwelteigenschaften oder Zusammenführung bereits in der Plattform vorhandener Umweltsensordaten zur Weiterleitung durch die Kommunikationseinheit ggf. mit einer vorherigen (Vor-)Auswertung kann dann an der Plattform als Zusatzmodul befestigt werden. Hierzu bietet sich ein Aufsteckmodul, ein Anclipsmodul oder ähnliches an.

Dies wird durch das Sitz- oder Liegemöbel erreicht, das die entfernt angeordnete Serviceeinheit zur einfachen und raschen Interaktion mit der dieses Möbelstück regelmäßig als Hauptlebensmittelpunkt nutzenden Person trägt. Die Serviceeinheit hat hierzu einen Monitor und eine Eingabeeinheit und ist zur drahtlosen Kommunikation mit dem autonomen Haushaltsgerät eingerichtet. Im Fußbereich des Sitz- oder Liegemöbels ist eine Ladestation zur Andockung und Aufladung des autonomen Haushaltsgerätes mit elektrischer Energie angeordnet. Damit bildet das Sitz- oder Liegemöbel (Sessel, Stuhl oder Pflegebett) nicht nur den Hauptlebensmittelpunkt der zu betreuenden Person, sondern auch die Zentrale für das zur Betreuung genutzte autonome Haushaltsgerät. Durch die erfindungsgemäße Anordnung der Ladestation im Fußbereich dieses Möbelstücks ist es für die zu betreuende Person und Besucher wenig auffällig positioniert und in einem Raumbereich betriebsbereit parkbar, an der die zu detektierenden Anzeichen für eine Hilfsbedürftigkeit mit der größten Wahrscheinlichkeit auftreten. Die Wege zur Umgebungsdetektion mit Hilfe der Umgebungssensoren sind damit optimal verkürzt.

Die Ladestation kann gleichzeitig als Dockingstation zum Aufbau einer drahtgebundenen Datenverbindung mit der Kommunikationseinheit des autonomen Haushaltsgerätes ausgebildet sein. Hierzu haben die Ladestation und das autonome Haushaltsgerät elektrische Kontakte, die beim Ankoppeln des autonomen Haushaltsgerätes an die Ladestation in Kontakt miteinander treten. Damit kann ein im Vergleich zur drahtlosen Kommunikation wesentlich abgesicherter Datenaustausch gewährleistet werden.

Das autonome Haushaltsgerät kann eine Kamera haben, die mit der Kommunikationseinheit zur Übertragung von Umgebungsbildern an die Serviceeinheit eingerichtet ist. Mithilfe dieser Kamera kann z. B. bei erkannter Hilfsbedürftigkeit eine detailliertere Bildinformation an Pflege- und Hilfskräfte übermittelt werden. Die Kamera kann auch als Umgebungssensor genutzt werden, um bei Bedarf eine erkannte Hilfsbedürftigkeit zu verifizieren oder anhand der aufgenommenen Bilder durch Muster- oder Bildervergleich zu erkennen.

Das autonome Haushaltsgerät kann mit Hilfe der entfernt angeordneten Serviceeinheit oder von einer damit vernetzten Kommandoapplikation fernsteuerbar ausgebildet sein. Damit lassen sich ferngesteuerte Überwachungsfahrten durch Pflegepersonal durchführen, ohne dass die Wohnung der zu betreuenden Person besucht und betreten werden muss und die Person durch einen solchen Betreuungsbesuch ggf. gestört wird. Die zu betreuende Person kann auf diese Weise auch in Kontakt zu einer Pflegeperson aufnehmen und auf schnelle und effiziente Weise Hilfe erhalten, wobei die Intensität der ferngesteuerten Pflege und Kontaktaufnahme steuerbar ist. Es wird somit eine Überwachung ohne physische Anwesenheit von Pflegepersonal ermöglicht.

Die Kamera kann dabei ferngesteuert ausrichtbar und/oder fokussierbar sein. Hierzu kann die Kamera Aktoren zur Änderung des Schwenkwinkels, Nickwinkels und/oder des Zoomverhältnisses haben. Damit ist es denkbar, dass Befehle, die von einer entfernten Serviceeinheit eingegeben werden, die Kamera bei erkannter Hilfsbedürftigkeit verschwenkt wird, um die Lage in der Umgebung der hilfsbedürftigen Person zu ermitteln und auf dieser Grundlage einen Hilfseinsatz zu koordinieren.

Das Haushaltsgerät kann mindestens ein Mikrofon zur Erfassung von Schallsignalen haben. Die Personenhilfseinheit kann dann mittels erfasster Schallsignale zur gesteuerten Fortbewegung zu der Schallquelle hin und/oder zur Erkennung von der Hilfsbedürftigkeit in Abhängigkeit eines Vergleichs erfasster Schallsignale mit vordefinierten Schallmustern eingerichtet sein. Mithilfe des Mikrofons gelingt es, dass die zu betreuende Person einen Hilferuf an das autonome Haushaltsgerät absetzt. Das autonome Haushaltsgerät ist dann in der Lage, die Hilfsbedürftigkeit zu verifizieren und z. B. mittels Mikrofons und Kamera eine Kommunikation zwischen entfernt verfügbaren Hilfspersonal über die entfernte Serviceeinheit mit der hilfsbedürftigen Person aufzubauen. Dabei ist zur akustischen Kommunikation vorteilhaft auch noch mindestens einen Lautsprecher in dem autonomen Haushaltsgerät integriert, um eine bidirektionale Audio-Kommunikationsverbindung zwischen der hilfsbedürftigen Person und der entfernten Serviceeinheit zu ermöglichen.

Mindestens ein Umgebungssensor des Haushaltsgerätes kann zur Detektion von Eigenschaften von mit der Bodenreinigungs- oder Rasenmäheinheit aus der Umgebung aufsammelbaren Elementen eingerichtet sein. So ist es beispielsweise möglich mithilfe von Gassensoren, chemischen Sensoren oder optischen Sensoren zu erkennen, ob Medikamente, Alkohol, Blut, Urin oder andere Körperexkremente mithilfe des Haushaltsgerätes aufgefangen wurden. Das Erkennen solcher Eigenschaften ist dann ein Hinweis für eine Hilfsbedürftigkeit. So ist das Auffinden von Alkohol ein Zeichen für das Verschütten von flüssigen Medikamenten, die in der Regel auf Alkoholbasis zubereitet sind, und damit ein Hinweis auf die Gefahr einer vorschriftswidrigen Medikamenteneinnahme. Bevorzugt sind die Umgebungssensoren und die nachgelagerten Detektionsroutinen ausgebildet, um pharmazeutische Mittel anhand typischer Arzneiträgerstoffe oder pharmazeutischer Hilfsstoffe zu erkennen. Dies können Füllstoffe (z. B. Lactose, Cellulose, Stärke, Saccharose, Paraffin, Hartfett, Polyethylenglykoel, Polyethylenoxide), Lösungs- oder Befeuchtungsmittel (z. B. Wasser, Ethanol, Isopropanol), Emulgatoren (z. B. Cetylstearylalkohol, Gylcerolmonostearat, Lecithin, Fettsäureester des Sorbitans, des Polyoxyethylensorbitans (Polysorbate), des Polyoxyethylens, Polyoxyethylenfettalkoholether), Lösungsvermittler oder Netzmittel (z. B. Polyethylenglykole, Polyethylenoxide, Polysorbate), Puffer (z. B. Natriumdihydrogenphosphat, Natriumhydrogencarbonat, Calciumhydrogenphosphat, Trometamol), Verdickungs- und Bindemittel (z. B. Stärken, Guaran, Xanthan, Alginat, Carrageen, Pektin, Traganth, Polyacrylsäuren, Polyvinylpyrrolidon; hochdisperses Siliciumdioxid, substituierte Celluloseether (Methylcellulose, Ethylcellulose, Hydroxypropylcellulose, Hydroxypropylmethylcellulose, Carboxymethylcellulose), Umhüllungsmittel (z. B. Saccharose, Gelatine, Gelatinepolysuccinat, Polyacrylate, Ethylcellulose, Methylcellulose), Zerfallsbeschleuniger und Sprengmittel (z. B. Stärken, Croscarmellose, Natriumhydrogencarbonat in Kombination mit Citronensäure), Gleit- und Schmiermittel und Fromtrennmittel (z. B. Polyethylenglykole, Polyethylenoxide, Talkum, Magnesiumstearat), Fließregulierungsmittel (z. B. hochdisperses Siliciumdioxid), Antioxidantien (z. B. Butylhydroxytoluol, all-*rac*-α-Tocopherol), Konservierungsstoffe (z. B. PHB-Ester, Benzalkoniumchlorid, Benzylalkohol, Thiomersal), Süßungsmittel und Geschmackskorrigientien (z. B. Saccharose, Sorbit, Süßstoffe wie etwa Saccharin-Natrium und Cyclamat; Aromen) oder Resorptionsbeschleuniger (z. B. Dimethylsulfoxid) sein.

Umgebungssensoren können beispielsweise in Borsten oder Lappen einer Bodenreinigungseinheit integriert sein. Unter einer Bodenreinigungseinheit wird nicht nur eine zur Reinigung einer horizontalen Ebene geeignete Einheit verstanden. Denkbar ist auch z. B. eine Glasoder Wandreinigungsfunktion für vertikale Ebenen, wobei ein solches Haushaltsgerät zur Patientenüberwachung aufgrund des möglichen Blickwinkels schräg von oben nützlich ist, wenn z. B. ein Pflegebett neben einem Fenster steht.

Denkbar ist in Verbindung mit einer Bodenreinigungseinheit die Nutzung von Nachweisflüssigkeiten, mit denen die Borsten oder Lappen manuelle oder automatisch getränkt werden. Durch Integration optischer Sensoren in der Nähe der Borsten oder Lappen kann dann die Einfärbung der Borsten oder Lappen untersucht und anhand charakteristischer Farbwerte oder Farbspektren auf Umgebungseigenschaften geschlossen werden.

Auf solche Eigenschaften kann beispielsweise beim Überschreiten vorgegebener Schwellwerte geschlossen werden. Denkbar ist auch eine Korrelation mit anderen erfassten Eigenschaften oder eine Erkennung von Hilfsbedarf bei über einen Zeitraum auftretender Häufung solcher Vorkommnisse. Dabei ist es hilfreich, zur Pflegedokumentation solche Ereignisse zu protokollieren, um es als Pflegetagebuch ausdrucken zu können oder eine offene Schnittstelle zu haben, so dass eine Anbindung an eine Pflegedokumentation eines Pflegeanbieters möglich ist.

Ein als Saugroboter ausgestaltetes autonomes Haushaltsgerät kann einen transparenten Auffangbehälter mit einer Grobfiltereinheit haben, die mit der Bodenreinigungseinheit verbunden und zum Auffangen von auf dem Boden liegenden Medikamenten geeignet ist. Damit gelingt es durch Sichtprüfung schnell festzustellen, ob sich im Auffangbehälter Medikamente gesammelt haben, die auf eine nicht ordnungsgemäße Medikamenteneinnahme und damit einer Hilfsbedürftigkeit schließen lassen.

Die Personenhilfseinheit kann zur Aktivierung einer Hilfeeinheit eingerichtet sein, die mindestens eine Kamera und ein Mikrofon umfasst. Die Aktivierung erfolgt, wenn mittels mindestens eines tastenden Sensors, Gassensors, Lichtsensors und/oder Schallpegelsensors eine Hilfsbedürftigkeit erkannt wurde. Auch hier können beispielsweise vordefinierte Schwellwerte als Kriterium zur Aktivierung herangezogen werden. So kann mithilfe eines tastenden Sensors beispielsweise festgestellt werden, ob eine Person am Boden liegt. Mithilfe von Gassensoren lassen sich auffällige Zusammensetzungen der Umgebungsluft, wie beispielsweise Sauerstoffmangel oder ein erhöhter Uringehalt feststellen. Ein reduzierter Sauerstoffmangel in der Umgebungsluft zeigt, dass die zu betreuende Person nicht mehr in der Lage ist, selbsttätig für eine regelmäßige Lüftung zu sorgen. Eine erhöhte Urinkonzentration in der Luft ist ein Zeichen, dass beispielsweise Inkontinenz vorliegt oder Windel gewechselt werden müssen.

Die Personenhilfseinheit kann mindestens eine Schnittstelle zur Anbindung von Personen-Vitaldatensensoren und zur Auswertung und/der zur Übertragung von mit den Personen-Vitaldatensensoren erfassten Sensordaten über die Kommunikationseinheit an eine entfernte Serviceeinheit eingerichtet sein. Dies hat den Vorteil, dass die mit dem autonomen Haushaltsgerät gekoppelte Personenhilfseinheit weiterhin an zusätzliche Geräte vorzugweise drahtlos ankoppelbar ist, mit den Personen-Vitaldaten aufgenommen werden. Dabei kann es sich beispielsweise um Personenwagen oder z. B. am Handgelenk oder um den Brustumfang zu tragende Puls- und Herzrhythmusmessgeräte, Blutzuckermessgeräte, Körpertemperaturmessgeräte und ähnliches handeln. Die Datenübertragung erfolgt dabei vorzugsweise drahtlos über geeignete Funkkommunikationsschnittstellen. Mithilfe dieser Personen-Vitaldatensensoren kann zudem noch eine Korrelation mit den Umgebungseigenschaften vorgenommen werden, die mithilfe der Umgebungssensoren des autonomen Haushaltsgeräts erfasst werden. Damit kann die Qualität der Erkennung der Hilfsbedürftigkeit verbessert werden.

Mit der Serviceeinheit des Sitz- oder Liegemöbels können je nach Tageszeit oder Verhaltensmustern der zu betreuenden Person unterschiedliche Stufen der Sensibilität des autonomen Haushaltsgerätes vorgegeben werden. So kann die Serviceeinheit das autonome Haushaltsgerät beispielsweise für den Zeitbereich einer geplanten Medikamenteneinnahme in erhöhte Alarmbereitschaft versetzen, so dass das autonome Haushaltsgerät in diesem Zeitbereich verstärkt die Umgebung des Sitz- und Liegemöbels und andere mit der Medikamenteneinnahme zusammenhängende Raumbereiche nach heruntergefallenen Medikamenten absucht. Die Erkennungsschwelle des autonomen Haushaltsgerätes kann für Zeitbereiche, in denen Pflegeereignisse stattfinden, auch geändert werden.

Die Personenhilfseinheit kann zur drahtlosen Öffnung von Türschließeinheiten ausgebildet sein, wenn eine Hilfsbedürftigkeit erkannt würde. Hierzu kann die Personenhilfseinheit durch eine geeignete Parametrierung ermöglicht werden, um mithilfe einer Funk-Kommunikationseinheit Funktürschlösser zu entriegeln, um somit Hilfspersonen den Zutritt zu den ansonsten von außen geschlossenen Räumen zu ermöglichen. Dies ist z. B. für ansonsten von innen abschließbare Sanitärräume hilfreich.

Das autonome Haushaltsgerät kann weiterhin eine Warneinheit zur Ausgabe von optischen und/oder akustischen Warnsignalen bei mittels des mindestens einen Umgebungssensors erfasster Annährung einer Person an das Gerät haben. Damit wird die Gefahr reduziert, dass die pflegedürftigen Personen versehentlich auf das autonome Haushaltsgerät treten und stürzen. Die Warneinrichtung kann beispielsweise so eingerichtet sein, dass das optische und/oder akustische Signal umso deutlicher wird, je näher die Umgebungssensoren an Menschen und Tiere kommen.

Die Serviceeinheit ist bevorzugt in eine bewegbare Armlehne oder eine bewegbare Ablage (z. B. Tisch) des Sitz- oder Liegemöbels integriert. Die Armlehne oder Ablage kann dabei vom sitzenden Benutzer beispielsweise durch Verschwenken oder Verbiegen in eine Position gebracht werden, in welcher der Benutzer den Bildschirm der in der Armlehne oder der Ablage integrierten Serviceeinheit gut erfassen und im Falle eines berührungsempfindlichen Bildschirms (Touch-Screen) auch bedienen kann. Die Armlehne bietet damit zugleich eine Auflagefläche für die Arme und eine Aufnahme für die Serviceeinheit, insbesondere für den Monitor der Serviceeinheit, um die dort beweglich aufgenommene Serviceeinheit in das Gesichts- und Bedienfeld des Benutzers zu bringen. Diese Anordnung bietet vor allem bei sehbeeinträchtigten Personen einen Vorteil, da die Positionierung an der Armlehne auch optimal in Sicht- und Augennähe ist.

Die Integration der Serviceeinheit in eine Armlehne oder Ablage, d. h. den integralen Einbau in die Armlehne oder Ablage, hat im Vergleich zu dem Anbau an einem von der Armlehne oder Ablage entkoppelten Stativ den Vorteil, dass die Serviceeinheit unscheinbar ist. Eine an einem Stativ montierte Serviceeinheit kann auch leichter abgebaut und verlegt werden, wohingegen die in die Armlehne oder Ablage integrierte Serviceeinheit dort in jedem Falle verbleibt und damit zuverlässig verfügbar ist. Die zur Personenüberwachung und Pflege benötigte Technik wird auf diese Weise in ein vorhandenes (ggf. mobiles) Sitz- oder Liegemöbel integriert und steht am Lebensmittelpunkt der Person zuverlässig zur Verfügung.

Vorteilhaft ist es, wenn das Sitz- und Liegemöbel einen Getränkehalter hat, der mit Sensoren zur Überwachung des Getränkekonsums des Patienten aus dem im Getränkehalter positionierbaren Gefäß gekoppelt ist. Damit kann die Serviceeinheit eingerichtet sein, bei unzureichender Getränkeaufnahme eine Erinnerung bspw. über einen Lautsprecher auszugeben. Der Getränkehalter kann ein thermisches Element zur Warmhaltung und/oder Kühlung des im Getränkehalter angeordneten Gefäßes bzw. seines Inhalts haben.

Es ist weiterhin vorteilhaft, wenn mindestens ein Mikrofon und/oder Lautsprecher in das Sitzoder Liegemöbel eingebaut und mit der Serviceeinheit verbunden ist. Das Sitz- und Liegemöbel kann zudem darin integrierte Vitaldatensensoren haben.

Das Sitz- und Liegemöbel kann stationär sein. Es kann aber auch mobil, d. h. verfahrbar sein. Hierzu bietet sich an, wenn das Sitz- oder Liegemöbel Rollen hat, die arretierbar oder versenkbar sind, um zwischen einem mobilen verfahrbaren Zustand und einem sicheren stationären Zustand wechseln zu können.

Das Sitz- und Liegemöbel mit seiner Serviceeinheit bildet die Zentrale, in der die weiteren Pflegehilfsmittel, wie insbesondere das autonome Haushaltsgerät und das zur Medikamentengabe eingerichtete Haushaltsgerät, zusammengeführt und miteinander vernetzt werden. Es bildet hierfür sozusagen eine Datenbrücke und zentrale Logistikeinheit, um beispielsweise als Medikamentenwecker die Medikamenteneinnahme mit Hilfe des Haushaltsgerätes zu steuern und mit Hilfe des mobilen autonomen Haushaltsgerätes die Medikamenteneinnahme zu überwachen. Die Serviceeinheit des Sitz- oder Liegemöbels kann mit dem Haushaltsgerät kommunizieren, um die Medikamentengabe zu steuern oder zumindest Informationen über Medikamentenbeimischung in Lebensmittel abzurufen, um auf dieser Grundlage durch Ansteuerung oder Abfrage des autonomen Haushaltsgerätes und/oder weiterer Vitaldatensensoren die korrekte Medikamenteneinnahme zu überwachen und ggf. bei Abweichungen von vorgegebenen Umgebungs- und/oder Vitaldaten eine Hilfebedürftigkeit zu erkennen. Die Kommunikation der Serviceeinheit des Sitz- oder Liegemöbels mit dem autonomen Haushaltsgerät ermöglicht bedarfsgerechte Überwachungen der Umgebung der Person, um durch die Mobilität des autonomen Haushaltsgerätes dem Tagesablauf der zu betreuenden Person folgen zu können und diese im Hinblick auf eine etwaige Hilfebedürftigkeit unauffällig zu überwachen. Dabei können Vitaldatensensoren eine weitere Überwachung durch die Serviceeinheit und der damit vernetzten Pflegehilfsmittel auslösen. Durch die Interkommunikation der Pflegehilfsmittel lassen sich valide Informationen zur Hilfebedürftigkeit der Person ableiten, die über die einzelnen Daten des autonomen Haushaltsgerätes oder des Haushaltsgerätes hinausgehen. So kann aufgrund von sensierten Daten des Haushaltsgerätes, das zur Beimischung von Medikamenten in Lebensmitteln eingerichtet ist, eine Überwachungsfahrt durch das autonome Haushaltsgerät ausgelöst werden, um damit weitere Informationen zur Hilfebedürftigkeit zu erhalten.

Denkbar ist auch die weitere Vernetzung mit anderen Pflegehilfsmitteln, wie beispielsweise mit Vitaldatensensoren von Toiletten, Geschirr, Besteck oder ähnlichem, die zur Detektion von Zuständen der zu betreuenden Person eingerichtet sind und/oder mit Sensoren an Küchengeräten, wie Kühlschränken (z. B. zur Ermittlung der Haltbarkeit von Lebensmitteln oder deren Entnahme), welche ebenfalls Indikatoren zur Hilfsbedürftigkeit liefern. Durch die Gesamtschau dieser Indikatoren gelingt es insbesondere mit Hilfe des mobilen autonomen Haushaltsgerätes, die aus der Zusammenschau der Daten gewonnenen Informationen zur Hilfebedürftigkeit zu verifizieren, Zusammenhänge zu evaluieren und Ursachen zu erkennen.

Das autonome Haushaltsgerät kann auf diese Weise durch die Serviceeinheit angesteuert werden, um mit adaptierter Sensibilität Umgebungseigenschaften zu erfassen, mit denen eine Hilfebedürftigkeit der zu betreuenden Person noch sicherer erkannt und ggf. validiert werden kann.

Das zur Medikamentengabe eingerichtete Haushaltsgerät kann ein zur Zubereitung von Lebensmitteln mit einem Gefäß ausgebildetes Haushaltsgerät sein, das eine Dosiereinheit hat, die mit dem Gefäß verbunden ist. Das zur Medikamentengabe ausgebildete Haushaltsgerät ist vorgesehen und geeignet, auch unabhängig von der Medikamentengabe ein Lebensmittel für den Benutzer bereitzustellen und unterscheidet sich damit elementar von aus der US 2016/0058245 A1 bekannten Geräten, die ausschließlich zur Zubereitung von Energiedrinks mit Nahrungsergänzungsmitteln ausgebildet sind.

Die Dosiereinheit hat ein Dosiergefäß zur Aufnahme von Medikamenten. Eine Steuerungseinheit ist zur Ansteuerung der Dosiereinheit vorgesehen, die zur vorgegebenen Beimischung von Medikamenten aus dem Dosiergefäß zu dem im Gefäß befindlichen Lebensmittel in Abhängigkeit von der Zubereitung eines Lebensmittels eingerichtet ist.

Für die kontrollierte Medikamentengabe wird ein vom Patient im Tagesablauf regelmäßig genutztes Haushaltsgerät verwendet, wobei die zu verabreichenden Medikamente bei der üblichen Zubereitung des Lebensmittels diesem beigemischt werden, damit erfolgt die Medikamentenabgabe automatisierbar zusammen mit der Lebensmittelzubereitung und Lebensmitteleinnahme, ohne dass gesonderte Geräte oder Vorrichtungen zur Medikamentenzuteilung erforderlich sind. Diese Kombination der Dosiereinheit zur Medikamentenbeimischung mit einem Haushaltsgerät eignet sich für Ein-Personen-Haushalte, wie sie regelmäßig bei der Seniorenpflege anzutreffen sind. Die Dosiereinheit kann als separates Teil auf das Haushaltsgerät aufsetzbar oder in das Haushaltsgerät integriert sein.

Das zur Medikamentengabe eingerichtete Haushaltsgerät hat vorzugsweise eine Rühreinheit in dem Gefäß, die dann zur Vermischung des eindosierten Medikaments in das Lebensmittel gleich mitbenutzt werden kann.

Das zur Medikamentengabe eingerichtete Haushaltsgerät kann eine mit dem Gefäß gekoppelte Heiz- und/oder Kühleinheit haben. Damit kann das zuzubereitende Lebensmittel zum Garen erhitzt oder abgekühlt werden. Mit Hilfe dieser Heiz- und/oder Kühleinheit kann durch geeignete Ansteuerung über die Steuerungseinheit eine für das Eindosieren von Lebensmitteln und insbesondere von Medikamenten günstige Temperatur eingestellt werden. Das Dosiergefäß kann mehrere voneinander getrennte Medikamentenaufnahmebereiche haben. Die Dosiereinheit ist zur gesteuerten Beimischung ausgewählter Medikamente aus den Medikamentenaufnahmebereichen eingerichtet. Damit ist es möglich, eine komplexere Medikamentenverordnung bedarfsgerecht umzusetzen, bei der verschiedene Medikamente in den einzelnen Medikamentenaufnahmebereichen vorbereitet und jeweils bedarfsgerecht zur gewünschten Zeit oder zu passenden Lebensmitteln beigemischt werden.

Die Dosiereinheit kann ein Mahlwerk zur Zerkleinerung von Medikamenten haben, die in fester Form vorliegen. Auf diese Weise können Tabletten oder Kapseln zerkleinert und derart aufbereitet in das Lebensmittel eingemischt werden.

Besonders vorteilhaft ist es, wenn die Medikamente bereits in Medikamentengaben dosiert in individuell für den Patienten zusammengestellte Verpackungen bereitgestellt werden. Diese mindestens eine Verpackung wird dann der Dosiereinheit zugeführt, die in Abhängigkeit von der vorgegebenen Einnahmezeit für ein Medikament eine entsprechend hierzu bereitgestellte Einzelverpackung oder einen Verpackungsabschnitt öffnet und in ein Lebensmittel eindosiert. Eine solche Verpackung kann beispielsweise ein Blister sein, der in Blisterabschnitten individuell eingepackte Medikamente tageszeitabhängig bereitstellt. Ein Blisterstreifen kann dann durch die Dosiereinheit mit Förderelementen bewegt werden, um je nach Tageszeit einen Blisterabschnitt in den Eingriffsbereich einer Eindosiereinheit zu bringen, welche den Blisterabschnitt öffnet und den flüssigen, zähflüssigen oder festen Inhalt aus dem Blisterabschnitt in das Gefäß zu bringen. Hierzu kann der Blisterabschnitt ggf. durchspült werden. Denkbar ist auch, dass eine Mahleinheit zwischen dem Blisterabschnitt und dem Gefäß angeordnet ist, um das Medikament vor der Eindosierung zu zermahlen.

Denkbar ist aber auch, dass die Medikamente tageszeitabhängig ist einzelnen Kapseln bereitgestellt werden, die dann von der Dosiereinheit zum Eindosieren der darin enthaltenen Medikamente geöffnet werden. Diese separaten Kapseln können auch wie ein Blisterstreifen an einem gemeinsamen Träger angeordnet sein. Auch hier sind die Medikamente individuelle und portioniert in Blisterabschnitten in steriler Weise gelagert und können von der Dosiereinheit bedarfsweise gemäß einem für den Patienten individuell vorgegebenen Medikamenten-Zeitplan eindosiert werden.

Die Dosiereinheit kann alternativ oder zusätzlich hierzu zum Einleiten von Dosierflüssigkeit in das Dosiergefäß ausgebildet sein, um das in das Gefäß einzudosierende Medikament, das sich im Dosiergefäß befindet, in der Dosierflüssigkeit aufzulösen und/oder mit der Dosierflüssigkeit zu vermischen, um dann diese Dosierflüssigkeit in das Lebensmittel im Gefäß einzubringen. Ein Medikament, das in Flüssigform im Dosiergefäß bereitgestellt wird, kann auf diese Weise mit Hilfe der Dosierflüssigkeit aus dem Dosiergefäß herausgespült und mit der Dosierflüssigkeit in das Lebensmittel eingemischt werden. Die Dosierflüssigkeit selbst kann aber auch schon das im Gefäß zuzubereitende Lebensmittel sein.

Wenn als Dosierflüssigkeit Wasser genutzt wird, dann hat dies den Vorteil, dass das Dosiergefäß beim Eindosieren des Dokuments gespült wird.

Die Dosiereinheit kann mit einem Deckel des Gefäßes verbunden oder an dem Deckel des Gefäßes angeordnet sein. So ist es möglich, ein handelsübliches Haushaltsgerät zu nutzen, dessen Deckel gegen einen mit Dosiergefäß und Dosiereinrichtung versehenen Deckel ausgetauscht wird. Die Dosiereinheit kann dabei mindestens teilweise durch eine elektronische Steuerungseinheit des Haushaltsgerätes realisiert werden. Denkbar ist aber auch, dass z. B. in dem Deckel des Gerätes eine separate elektronische Dosiereinheit eingebaut ist, mit der die Medikamentenbeimischung bedarfsgerecht gesteuert werden kann. Dies kann zeit- und ereignisgesteuert sein. So kann beispielsweise mit Hilfe von Sensoren im Deckel des Gefäßes erkannt werden, dass das Haushaltsgerät im Betrieb ist. In Abhängigkeit von der Uhrzeit kann in diesem Betriebszustand dann ggf. eine vorprogrammierte Medikamentenbeimischung gestartet werden.

Vorteilhaft ist es, wenn eine Datenschnittstelle zum Einlesen von Rezeptdaten vorhanden ist, die Verabreichungszeiten, Zeitintervalle, Mengen und/oder Inhalte von zu verabreichenden Medikamenten umfassen. Die Steuereinheit ist dann zum Eindosieren der Medikamente in Abhängigkeit von den über die Datenschnittstelle eingelesenen Rezeptdaten eingerichtet. Eine solche Datenschnittstelle kann als Verbindungsstecker zu einer Datenspeicherkarte (z. B. SD-Karte), als Funkdatenübertragungseinheit im Nahbereich z. B. über WLAN oder Bluetooth oder im Fernbereich über Mobilfunkkommunikation, als Leseeinheit für einen Transponder (RFID) und/oder als optischer Datencodeleser (z. B. für OCR- oder Barcode) haben. Mit Hilfe einer solchen Datenschnittstelle ist es sehr einfach möglich, die Rezeptdaten zuverlässig von einem in elektronischer Form oder in Papierform vorliegenden Rezept auf die Dosiereinheit des Haushaltsgerätes zu übertragen. Am einfachsten ist es dabei, wenn die Medikamentenbeimischung als optisch lesbarer Code bereits im Rezept abgelegt ist, wobei diese Rezeptinformation zur Beimischung der Medikamente und zur Ansteuerung des Haushaltsgerätes dann nur noch über die Datenschnittstelle an das Haushaltsgerät übertragen werden müssen. Damit können Fehlereinflüsse reduziert werden.

Auch eine Blisterverpackung oder sonstige Medikamentenverpackung kann mit codierter Information, wie beispielsweise OCR-Codes, Barcodes oder RFID versehen werden. Das Haushaltsgerät hat eine zum Auslesen dieser Information eingerichtete Blisterinformations-Ausleseeinheit, um auf diese Weise sicherzustellen, dass nur das für das Haushaltsgerät freigegebene personenindividuelle Medikament zu den ggf. mit den Blisterabschnitten zusammenhängend abgelegten Zeitinformationen passenden Dosierungszeiten in das Lebensmittel eindosiert wird. Zudem kann eine solche Information als Steuerungsparameter für die Nachbehandlung des Medikamentes beispielsweise durch Zerkleinern genutzt werden.

Zudem ist eine Identitätsprüfung und ein Abgleich mit der Identitätsinformation an der Blisterverpackung möglich. Ein solcher Personenabgleich kann auch mit einem zusätzlichen Identitätssensor an dem Haushaltsgerät kombiniert werden. So kann das Haushaltsgerät einen Fingerprintsensor zur Erfassung der Fingerabdrücke der das Haushaltsgerät nutzenden Person haben, um dann nur bei Übereinstimmung mit freigegebenen Personendaten das personenindividuelle Medikament einzudosieren. Denkbar ist in diesem Zusammenhang auch eine Identitätsprüfung durch Funkdatenabgleich mit einer sogenannten Smart-Watch oder eines RFID-Transponders einer in der Nähe des Haushaltsgerätes befindlichen Person.

Die Medikamentenbereitstellung und Einnahme kann durch die Bereitstellung der Medikamente in personenindividuell zusammengestellten verblisterten Einheiten vereinfacht werden. Einzelpackungen verschiedener Medikamente in unterschiedlichen Packungsgrößen werden vermieden, die oftmals nur zum Teil aufgebraucht werden.

Das zur Medikamentengabe eingerichtete Haushaltsgerät kann ein Wasserkocher, ein Garautomat, ein Teeautomat, ein Kaffeeautomat, ein Küchenautomat, ein Wassersprudler oder ähnliches sein. Damit werden bereits vorhandene und täglich vom Patienten genutzte Haushaltsgeräte verwendet, um eine vorgegebene Medikamentengabe durch Beimischung bei der Lebensmittelzubereitung sicherzustellen, ohne dass der Patient die Medikamente bewusst zu vorgegebenen Zeiten einnehmen muss. Damit kann sichergestellt werden, dass auch pflegebedürftige und insbesondere demente Patienten ohne übermäßigen Betreuungsaufwand ihren Medikamentenplan einhalten.

Die Medikamentenbeimischung kann zusammen mit den Informationen über das zubereitete Lebensmittel abgespeichert werden und als Datensatz zur Pflegedokumentation abrufbar sein. Auf diese Weise kann nachverfolgt werden, welche Flüssigkeitsmenge, welche Energiemenge, welche Mengen an Fetten, Eiweißen und Kohlenhydraten sowie Vitaminen und dergleichen der Patient eingenommen hat und zu welchen Zeiten und Zeitabständen Medikamente beigemischt und dann offensichtlich eingenommen wurden.

Vorteilhaft ist, wenn das zur Medikamentengabe eingerichtete Haushaltsgerät zum Empfang von aktuellen Vitaldaten der zu behandelnden Person beispielsweise von Vitaldatensensoren der Person (z. B. Smart-Watch) eingerichtet ist. Solche Vitaldaten können beispielsweise der Blutdruck, der Puls und Pulsrhythmus, die Sauerstoffsättigung und der Körperwassergehalt sein. Die Dosiereinheit kann dann eingerichtet sein, um die Medikamentengabe in einem vorgegebenen Toleranzbereich in Abhängigkeit von solchen empfangenen Vitaldaten zu regeln.

Das zur Medikamentengabe eingerichtete Haushaltsgerät kann eine Alarmeinheit haben, um bei einem Ausbleiben der Lebensmittelentnahme aus dem Haushaltsgerät nach Zubereitung eine Alarmmeldung z. B. als Pflegenotruf an einen Pflegedienst und/oder an Angehörige abzusetzen. Zudem kann das Haushaltsgerät eingerichtet sein, um die Medikamentengabe zu protokollieren und online ggf. zusammen mit von Vitaldatensensoren empfangenen Vitaldaten des Patienten an medizinisches Personal zu übertragen. Denkbar ist auch, dass das Haushaltsgerät eingerichtet ist, den Medikamentenplan ferngesteuert über eine Datenschnittstelle durch den behandelnden Arzt zu ändern. Hierzu sollten zusätzliche Sicherheitsroutinen implementiert sein, wie bspw. Zertifikatsroutinen, um sicherzustellen, dass Eingriffe und Änderungen der Dosierung nur durch befugte Personen vorgenommen werden können.

Diese Routinen können in Verbindung mit einem Computerprogramm mit Programmcodemitteln realisiert werden, die zur Auswertung der Daten zu Umgebungseigenschaften und ggf. Vitaldaten und Erkennung der Hilfebedürftigkeit anhand dieser Daten ausgebildet sind, wenn das Computerprogramm auf einer Datenverarbeitungseinheit ausgeführt wird.

Die Erfindung wird nachfolgend anhand eines Ausführungsbeispiels näher erläutert. Es zeigen:
- Figur 1 -: Skizze eines autonomen Haushaltsgerätes mit entfernt angeordneter Serviceeinheit;
- Figur 2 -: Skizze eines Sitzmöbels mit integrierter Serviceeinheit und Ladestation für ein autonomes Haushaltsgerät;
- Figur 3 -: Skizze eines Haushaltsgerätes mit Dosiereinheit zur Beimischung von Medikamenten;
- Figur 4 -: Skizze des Haushaltsgerätes aus Figur 3 mit zur Beimischung von Medikamenten aus Blisterverpackungen eingerichteten Dosiereinheit;
- Figur 5 -: Skizze des Systems aus Sitzmöbel, autonomen Haushaltsgerät und Haushaltsgerät zur Lebensmittelversorgung unter Medikamentenbeimischung.

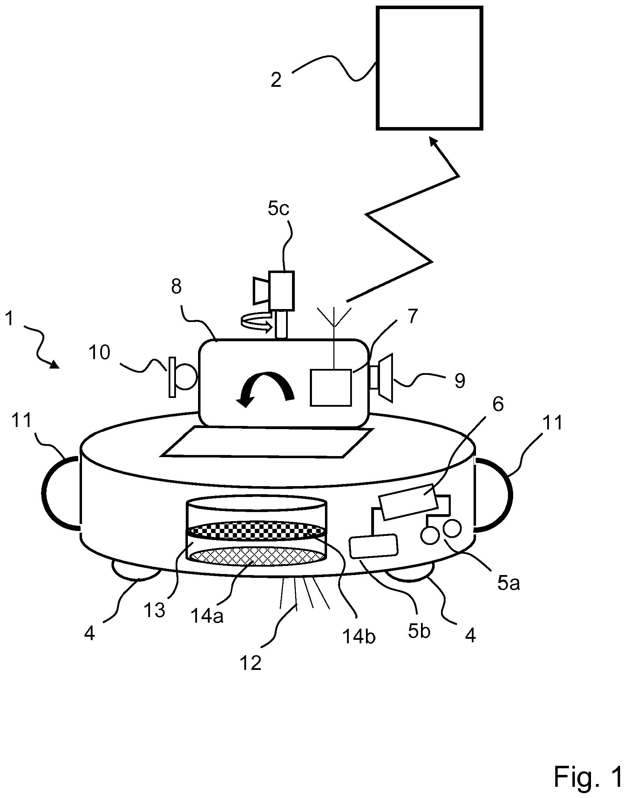

Figur 1 zeigt eine Skizze eines autonomen Haushaltsgerätes 1 mit einer entfernt angeordneten Serviceeinheit 2. Das autonome Haushaltsgerät 1 ist bspw. ein Saugroboter mit einer Plattform 3, die zur autonomen Bewegung ausgebildet ist. Hierzu hat die Plattform 3 Fortbewegungsmittel 4 bspw. in Form von mit Elektromotoren angetriebenen Rädern sowie Umgebungssensoren 5a, 5b zur Erkennung von Hindernissen. Solche Umgebungssensoren 5a können bspw. Ultraschall-Abstandssensoren oder taktile Sensoren 5b mit bei Berührung eines Hindernisses bewegbaren Tastern, optische Sensoren, wie bspw. Reflexionslichtschranken, Magnetfeldsensoren oder ähnliches sein. Denkbar ist auch, dass die Plattform 3 eine Ortungseinheit zur Positionsbestimmung der Plattform 3, wie bspw. ein Satellitennavigationsempfänger oder ein Inhouse-Ortungssystem hat.

Diese Umgebungssensoren 5a, 5b sind mit einer Steuerungseinheit 6 verbunden, die ihrerseits zur Ansteuerung der Fortbewegungsmittel 4 in Abhängigkeit von erkannten Hindernissen oder Wegstrecken eingerichtet ist.

Ein solches autonomes Haushaltsgerät 1 ist nun derart weitergebildet, dass es zur Erkennung der Hilfebedürftigkeit einer Person in Abhängigkeit der mit den Umgebungssensoren 5a, 5b, 5c detektierten Umgebungseigenschaften eingerichtet ist. Hierzu hat das autonome Haushaltsgerät 1 vorzugsweise weiterhin eine Kamera 5c als Umgebungssensor zur Aufnahme von Bildern der Umgebung. Diese Kamera 5c kann fest mit der Plattform 3 verbunden sein. Vorteilhaft ist aber, wenn die Kamera 5c wie durch den Pfeil angezeichnet verschwenkbar und/oder verdrehbar ist. Alternativ hierzu kann aber auch eine 380°-Kamera eingesetzt werden, um den gesamten Raumwinkelbereich der Umgebung der Plattform 3 beobachten zu können.

Das autonome Haushaltsgerät 1 hat weiterhin eine Kommunikationseinheit 7, die zum drahtlosen Aufbau einer Kommunikationsverbindung mit der entfernt angeordneten Serviceeinheit 2 hat.

Die Auswertung der Daten der Umgebungssensoren zur Erkennung einer Hilfsbedürftigkeit einer Person kann durch eine Recheneinheit des autonomen Haushaltsgerätes 1 erfolgen. Hierzu kann bspw. die Steuerungseinheit 6 geeignet programmiert sein oder eine zusätzliche Personenhilfseinheit 8 als Zusatzmodul mit der Plattform 3 verbunden werden. Denkbar ist aber auch, dass die Auswertung der Daten der Umgebungssensoren 5a, 5b, 5c nach drahtloser Übertragung zentral in der entfernt angeordneten Serviceeinheit 2 erfolgt, die dann zur Erkennung der Hilfebedürftigkeit einer Person in Abhängigkeit der Umgebungseigenschaften z. B. durch geeignete Programmierung eingerichtet ist.

Das autonome Haushaltsgerät 1 kann weiterhin mindestens einen Lautsprecher 9 und mindestens ein Mikrofon 10 haben, um eine Sprachkommunikationsverbindung zu der zu betreuenden Person aufzubauen. Diese Sprachkommunikationsverbindung kann dann drahtlos über die Kommunikationseinheit 7 mit der entfernt angeordneten Serviceeinheit 2 realisiert werden.

Die Aktivierung der Personenhilfseinheit 8 und insbesondere der Kamera 5c und des Mikrofons 10 kann zeitgesteuert in festgelegten Überwachungsintervallen oder zu vorgegebenen Überwachungszeiten erfolgen. Denkbar ist aber auch, dass die Personenhilfseinheit 8 ferngesteuert über die Serviceeinheit 2 aktiviert wird, wenn Betreuungspersonal ohne Besuch der zu betreuenden Person feststellen möchte, ob eine Hilfebedürftigkeit der zu betreuenden Person vorliegt.

Das autonome Haushaltsgerät 1 kann zudem Griffelemente 11 haben, die mit einer z. B. in der Steuereinheit 6 oder der Personenhilfseinheit 8 integrierten Vitaldatenmesseinheit gekoppelt sind. Wenn nun eine Person diese Griffelemente 11 berührt, dann können bspw. durch elektrische Impedanzmessungen Aussagen über den Wasser- und Fettgehalt getroffen werden. Möglich ist aber auch die Integration einer optischen Fingerpulseinheit zur Messung von Puls- und Sauerstoffsättigungswerten. Dies gelingt durch Reflexionsmessung in zwei Wellenlängenbereichen (Rot- und Infrarot-Licht) mittels hierzu ausgebildeter handelsüblicher Reflexionslichtschranken für diesen Zweck, die einfach in die Griffelemente 11 integriert werden können.

Das autonome Haushaltsgerät 1 hat zudem eine Bodenreinigungseinheit bspw. mit rotierbaren Borsten 12 oder Lappen.

Denkbar ist nun, dass auch dieser Teil der Bodenreinigungseinheit integrierte Umweltsensoren hat. So können bspw. Sensoren in die Borsten 12 oder Lappen integriert werden. Denkbar ist dabei, dass in der Plattform eine Einheit zur Befeuchtung der Borsten 12 oder Lappen mit einer Nachweisflüssigkeit für bestimmte zu detektierende Stoffe hat, wie bspw. Alkohol, Urin, Blut oder ähnlichem. Es kann dann durch optische Sensoren, wie bspw. in bestimmten Wellenlängenbereichen befindliche Fotodioden oder Fototransistoren oder eine Farbkamera sein. Damit kann dann auf einfache Weise geprüft werden, ob der zu überwachende Stoff in der Umgebung des autonomen Haushaltsgerätes 1 gefunden wurde, indem die Nachweisflüssigkeit umschlägt und seine Farbe ändert.

Denkbar ist aber auch, dass in die Borsten 12 oder Lappen elektronische Sensoren integriert werden, wobei dann eine Verdrahtung zur Steuerungseinheit 6 oder der Personenhilfseinheit 8 vorgenommen wird oder diese Umgebungssensoren drahtlos bspw. mittels UWB ausgelesen werden.

Wenn die Plattform 3 als Saugroboter ausgebildet ist, dann empfiehlt es sich, dass ein vorzugsweise transparenter Auffangbehälter 13 vorhanden ist, um vom Boden abgesaugte Partikel aufzufangen. Der Auffangbehälter 13 hat dann einen Grobfilter 14a, um größere Teilchen wie insbesondere Tabletten durchzulassen, und einen nachgeschalteten Feinfilter 14b. Die für die Erkennung einer potentiellen Hilfebedürftigkeit relevanten Tabletten, die die zu betreuende Person nicht ordnungsgemäß eingenommen, sondern auf dem Boden verloren hat, sammeln sich dann in dem Auffangbehälter 13 und sind dort zur Erkennung der Hilfebedürftigkeit verfügbar. Hierzu kann bspw. ein optischer Sensor oder eine Kamera mit dem Auffangbehälter 13 gekoppelt sein, um das Vorliegen von Tabletten und dergleichen zu detektieren.

In den Auffangbehälter 13 können auch noch weitere Umweltsensoren, wie bspw. Alkoholdetektoren, Urindetektoren und dergleichen eingebaut sein, um anhand der aufgesaugten Stoffe für eine Hilfebedürftigkeit charakteristische Eigenschaften zu detektieren.

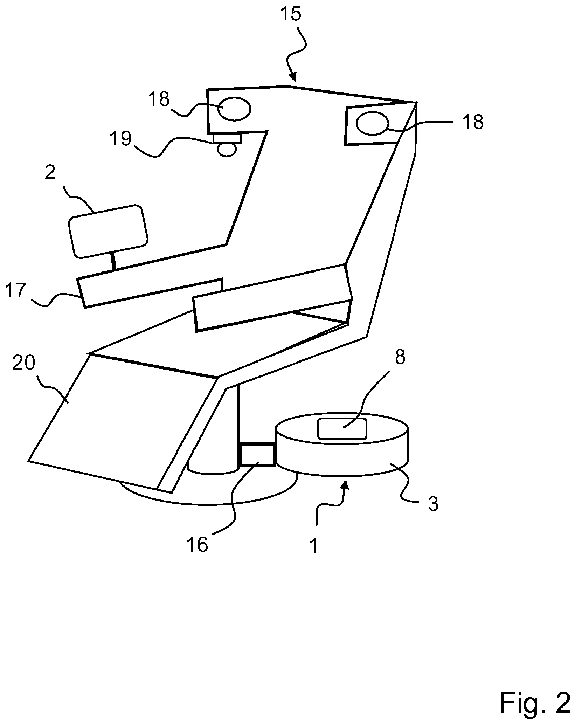

Figur 2 zeigt ein Sitzmöbel 15 für eine zu betreuende Person. Hierbei kann es sich um einen bequemen Ohrensessel handeln, aber auch um ein Pflegebett oder ähnliches. Das Sitzmöbel 15 hat nun in seinem Fußbereich eine Ladestation 16, an das das autonome Haushaltsgerät 1 andocken kann, um seine Energiespeicher nachzuladen. Dies kann bspw. durch elektrische Ladung von Akkumulatoren des autonomen Haushaltsgerätes 1 sein. Denkbar ist aber auch die Übergabe von Fest- oder Flüssigkraftstoffen.

Dadurch, dass die Ladestation 16 im Fußbereich des Sitzmöbels 15 angeordnet ist, wird das autonome Haushaltsgerät 1 in einer wenig auffälligen Position geparkt, die den üblichen Lebensmittelpunkt der zu betreuenden Person bildet. Damit werden die Fahrwege zu den für die Überwachung der Hilfebedürftigkeit vorrangigen Raumbereichen verkürzt. Die Überwachungsfrequenz für diesen Raumbereich ist erhöht, da das autonome Haushaltsgerät 1 die Umgebung des Sitzmöbels 15 regelmäßig aufgrund der räumlichen Nähe überprüfen kann. In diesem Bereich werden aber von der zu betreuenden Person regelmäßig die Medikamente eingenommen und durch verlorene Medikamente in der Nähe des Sitzmöbels 15 kann erkannt werden, dass die Gefahr einer unzureichenden Medikamenteneinnahme und damit eine Hilfebedürftigkeit besteht. Medikamente am Boden können bspw. über den Auffangbehälter 13 für Tabletten oder ähnliches und durch Alkoholsensoren für Flüssigmedikamente erkannt werden.

Das Sitzmöbel 15 ist zudem als Kommandozentrale für die zu betreuende Person ausgerichtet und hat hierzu eine schwenkbar, verschiebbar und/oder teleskopierbar an dem Sitzmöbel 15 angeordnete Serviceeinheit 2. Diese Serviceeinheit 2 kann bspw. ein Tablet-Computer mit einem Monitor und einer Eingabeeinheit sein. Diese Serviceeinheit 2 kann bspw. an einer verschwenkbaren Armlehne angeordnet sein, die umklappbar oder so bewegbar ist, dass die Serviceeinheit 2 von der auf dem Sitzmöbel 15 sitzenden Person gut bedient werden kann.

In das Sitzmöbel 15 können zudem Lautsprecher 18 und ein Mikrofon 19 integriert werden. Auf diese Weise kann ein Freisprechen über eine Kommunikationsverbindung mit nicht im Raum befindlichen Betreuungspersonen hergestellt werden.

Zur Bequemlichkeit kann eine klappbare Fußstütze 20 vorhanden sein, welche die Ladestation 16 und das darin befindliche autonome Haushaltsgerät 1 verdeckt und schützt.

Die Serviceeinheit 2 kann auch zur Lichtsteuerung der Wohnung der zu betreuenden Person eingerichtet sein. Vorzugsweise wirkt die Serviceeinheit 2 dabei mit dem autonomen Haushaltsgerät 1 zusammen, der durch Bewegungssensoren eine Bewegung der zu betreuenden Person erkennt und dann das Licht je nach den mittels Lichtsensoren erkannten Lichtverhältnissen oder nach der Tageszeit steuert, um die zu betreuende Person sicher durch die Wohnung zu leiten. Dabei ist es vorteilhaft, wenn das autonome Haushaltsgerät 1 bei einer Bewegung der Person aktiviert wird und die Person gegebenenfalls noch durch Voranfahren und Lichtsignale führt. Hierzu kann das autonome Haushaltsgerät 1 bspw. geeignet programmiert sein, um bei Nacht am Pflegebett geparkt zu werden und bei einem erkannten Aufstehen der zu betreuenden Person automatisch ferngesteuert das Licht in dem Raum anschaltet und optional in dieser Nachtsituation durch Lichtsignale die zu betreuende Person z. B. zu den Waschräumen führt und anschließend wieder zum Pflegebett zurückleitet.

So kann das autonome Haushaltsgerät 1 beispielsweise so programmiert sein, dass es mit der Lichtfunktion immer zwischen 6 bis 8 Uhr zu den Waschräumen leitet und anschließend in die Küche, da der Pflegebedürftige dort seine Medikamente nimmt. Diese erfasste Routine kann über das System später (re-)aktiviert werden, so dass das System von sich aus den Anstoß zur Durchführung (z. B. mittels Signal- oder Weckfunktion) dafür geben kann, wenn die Person in einem schleichenden Prozess, z. B. aufgrund einer Demenz, solche Routinehandlungen vergisst. Das autonome Haushaltsgerät 1 ist auf diese Weise zur Aktivitäts- und Inaktivitätserkennung mit Erinnerungsfunktion eingerichtet.

Dies ist durch situationsabhängige Programmierung von Betreuungsroutinen für ein autonomes Haushaltsgerät 1 relativ einfach realisierbar, da das autonome Haushaltsgerät 1 ohnehin eine selbstfahrende Plattform 3 hat.

Das autonome Haushaltsgerät 1 kann zudem eingerichtet sein, um in vorgegebenen Zeitintervallen die Umgebung abzufahren und zu überprüfen, ob sich die zu betreuende Person in einem hilfsbedürftigen Zustand befindet. Hierbei können auch Überwachungsfahrten zu Risikozeiten vorgegeben sein. Solche Risikozeiten können Zeitbereiche sein, in denen eine Medikamentengabe vorgesehen ist. So kann z. B. ein Haushaltsgerät, das zur Beimischung von Medikamenten in Lebensmittel eingerichtet ist, mit dem autonomen Haushaltsgerät gekoppelt sein, um in Zusammenhang mit der Beimischung von Medikamenten in ein Lebensmittel eine Überwachungsfahrt des autonomen Haushaltsgerätes im Raumbereich des anderen Haushaltsgerätes zu veranlassen und zu überwachen, ob Lebensmittel mit dem Medikament nicht auf den Boden gefallen oder verschüttet ist bzw. korrekt konsumiert wurde.

Durch eine tägliche Erfassung und Protokollierung der Aktivitäten durch das autonome Haushaltsgerät 1 kann eine Logik abgespeichert werden, so dass Abweichungen von der Routine (z. B. des Tagesablaufes der zu betreuenden Person) Indikatoren dafür sein können, dass eine Situation besteht in der Unterstützungsbedarf gegeben ist und damit eine Hilferuffunktion - als "Memoryfunktion" - aktiviert wird.

Für diese Überwachung werden dann die hierzu vorgesehenen und in die Privatsphäre gegebenenfalls eingreifenden Überwachungssensoren, wie die Kamera 5c und das Mikrofon 10 aktiviert. Dies kann durch Aufklappen der Personenhilfseinheit 8 erfolgen.

Vorteilhaft ist es weiterhin, wenn nicht nur das autonome Haushaltsgerät 1, sondern auch oder alternativ hierzu das Sitz- oder Liegemöbel 15 Handgriffe zur Messung von Vitaldaten hat, wie insbesondere Herz-/Kreislaufdaten der zu betreuenden Person. Solche Vitaldatensensoren können somit auch einfach in das Sitz- oder Liegemöbel 15 integriert und mit der Serviceeinheit 2 verbunden werden.

Denkbar ist es, wenn das autonome Haushaltsgerät 1 zur Ausführung einer vorgegebenen Hintergrundfunktion nach Auslösung einer Hilfe- oder Notruffunktion und Herstellung einer Kommunikationsverbindung eingerichtet ist. So kann z. B. das Abspielen vorprogrammierter oder voreingestellter Videos oder Musik ausgelöst werden, sobald ein Hilferuf aktiviert wurde, um so Stress-Situationen für die zu betreuende Person zu entschärfen. Viele Personen bekommen Panik, wenn sie z. B. stürzen. Die Wartezeit erscheint danach endlos und kann zusätzlichen Stress auslösen. Da Humor und Musik entspannt und eine Ablenkung zum Akutereignis schafft, ist es mit der Hintergrundfunktion möglich, die Wartezeit mental zu verkürzen und weitere Stress-Situation zu verringern.

Als technisches Assistenzsystem können aber auch andere Gegenstände genutzt werden, die von Menschen im täglichen Leben üblicherweise genutzt werden.

So ist es denkbar, einen Helm oder einen Hut mit Sensoren zur Sturzerkennung auszurüsten. Auch hier ist in diese Kopfbedeckung mindestens ein Sensor zur Sturzerkennung integriert. Dies kann beispielsweise ein Beschleunigungssensor, ein Magnetfeldsensor, ein Höhenmesssensor, ein Drucksensor oder eine Kombination solcher Sensoren sein. In die Kopfbedeckung sind dann auch noch eine Funkdatenübertragungseinheit und eine Steuereinheit integriert. Auch hier können wieder ein Ortungssensor zur Positionserkennung, eine Ladefunktion, ein Mikrofon, eine Kamera, aber auch Wärmesensoren integriert werden.

Wenn nun durch diese Sensoren ein Sturz der die Kopfbedeckung tragenden Person oder ein Hilfebedarf erkannt wird, kann eine Not- und Hilferuffunktion automatisch aktiviert werden. Denkbar ist auch, dass die Not- und Hilferuffunktion durch Sprachkommandos aktiviert werden. Mit Hilfe der Funkdatenübertragungseinheit kann in einem solchen Not- oder Hilfefall eine Sprach- und ggf. eine Bildkommunikation zu der Person hergestellt werden. Es können dann die Standortdaten, ein Foto oder Video und Vitaldaten über das technische Assistenzsystem an eine Notrufzentrale oder an vorbestimmte Angehörige übermittelt werden.

Sollte die Person nicht mehr vollständig kommunizieren, findet eine automatische Aktivierung des Hilfesystems statt, wobei eine automatische Bildübertragung an die vorgegebene Person erfolgen kann.

Mit Hilfe der in die Kopfbedeckung integrierten Sensoren kann beispielsweise ermittelt werden, ob die Vitalwerte sich im vorgegebenen Wertebereich bewegen. Auf diese Weise kann beispielsweise Schwindel oder Müdigkeit erkannt werden.

Die Steuerungseinheit der Kopfbedeckung kann zudem genutzt werden, um eine automatische Regelung der Ventilation bei übermäßiger Wärme, eine Zuluftreduzierung und ggf. Wärmezufuhr bei übermäßiger Kälte, den Windeinfluss und Regenschutz zur regeln. Mit Hilfe der Sensoren kann auch gemessen werden, welche Witterungsverhältnisse (Temperatur, Wärme, Wasser/Niederschlag oder Schnee etc.) herrschen. Eine solche Kopfbedeckung kann beispielweise beim Reiten, Ski fahren, Motorrad fahren, Paragleiten, Wassersport, E-Bike fahren etc. auch von Personen genutzt werden, die keine Pflegebetreuung benötigen. Dabei wird mit den Sensoren im aktivierten Zustand überprüft, ob die Kopfbedeckung auf den Boden fällt oder ob eine definierte Sturzgeschwindigkeit gemessen wird, um im "Standby-Status" in einen Hilferuf-/Notfallmodus umzuschalten.

Die Aktivierung dieser Überwachungsfunktion kann beispielsweise mit Hilfe eines Kinngurtes erfolgen, der mit einem Schalter ausgerüstet ist. Beim Schließen des Kinngurtes wird der Schaltkreis geschlossen und die Überwachungsfunktion aktiviert.

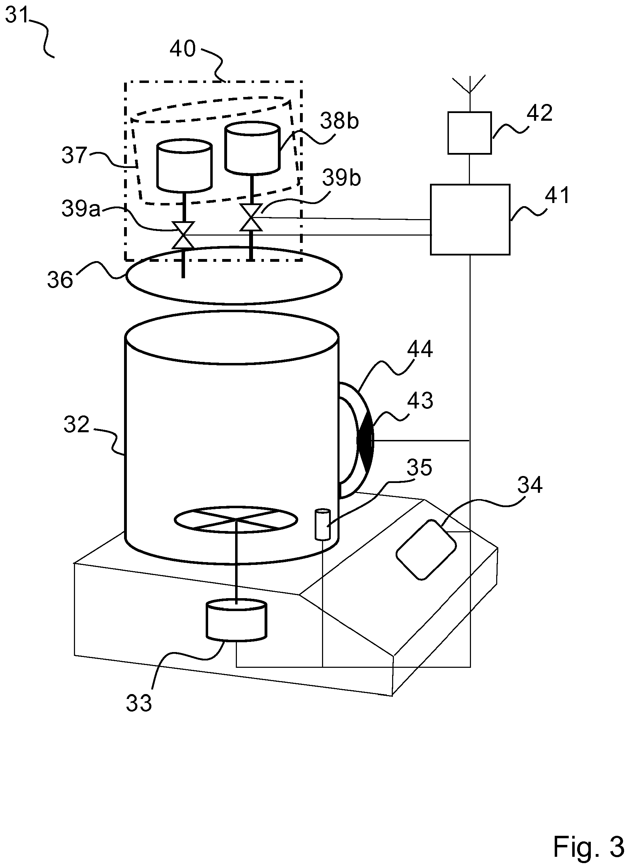

Figur 3 lässt eine Skizze eines zur Medikamentengabe eingerichteten Haushaltsgerätes 31 erkennen, das zur Zubereitung von Lebensmitteln ausgebildet ist. Hierzu hat das Haushaltsgerät 31 mindestens ein Gefäß 32 mit einer Rühreinheit 33, die zum Vermischen von den im Gefäß 32 enthaltenen Lebensmitteln vorgesehen ist. Das Haushaltsgerät 31 ist in dem Beispiel ein Küchenautomat, bei dem über eine Bedieneinheit 34 Rezepte ausgewählt und die schrittweise Zubereitung von Lebensmittel durch Eindosieren und Verrühren mit der Rühreinheit 33 und ggf. mit Erhitzen und Abkühlen mit einer Heiz- und/oder Kühleinheit 35 erfolgt. Das Gefäß 32 hat einen Deckel 36, der auf das Gefäß 32 aufsetzbar ist. In dem dargestellten Ausführungsbeispiel ist der Deckel 36 mit einem Dosiergefäß 37 verbunden. Das Dosiergefäß 37 kann beispielsweise fest mit dem Deckel 36 verbunden und/oder über ein Schlauchsystem mit dem Deckel 36 und damit mit dem Gefäß 32 im aufgesetzten Zustand gekoppelt sein. Das Dosiergefäß 37 kann mehrere Medikamentenaufnahmebereiche 38a, 38b haben, die jeweils über ein Dosierventil 39a, 39b mit einer Öffnung im Deckel 36 und damit mit dem Gefäß 32 verbindbar sind. Die Dosierventile 39a, 39b sind Teil der Dosiereinheit 40, um Medikamente in fester oder flüssiger Form bedarfsweise in das Gefäß 32 einzudosieren, die in dem Dosiergefäß 37 zur Beimischung vorbereitet sind. Hierzu ist eine Steuerungseinheit 41 vorgesehen, welche die Medikamente mit den Dosierventile 39a, 39b oder Dosierklappen oder ähnliches ansteuert, die Medikamente nach vorgegebenen Regeln zeit- und mengengesteuert bei der Zubereitung des Lebensmittels in dem Gefäß 32 beizumischen.

Das Programm zum Eindosieren der Medikamente kann der Steuereinheit 41 über die Eingabeeinheit 34 oder über eine andere Datenschnittstelle zugeführt werden. Eine solche Datenschnittstelle kann beispielsweise als Schacht mit Steckverbindern zur Aufnahme und Kontaktierung einer Datenspeicherkarte (SD-Karte oder ähnliches), eine Funkdatenübertragungseinheit 42 zur Datenfunkkommunikation, ein RFID-Leser zum Einlesen von Daten aus einem Funktransponder oder ein optischer Sensor zum Einlesen von Rezeptdaten beispielsweise über einen Barcode oder OCR-Code oder ähnliches sein.

In dem dargestellten Ausführungsbeispiel ist die Funkdatenübertragungseinheit 42 zusammen mit der Steuerungseinheit 41 eingerichtet, um auch eine Alarmmeldung an eine entfernte Pflegenotrufzentrale oder einen Angehörigen abzusetzen, wenn erkannt wurde, dass das automatisch zubereitete Lebensmittel nicht zu vorgegebenen Zeiten entnommen wurde. Die Entnahme des Lebensmittels kann sensiert werden, indem eine Entnahme des Gefäßes 32 aus der Plattform des Haushaltsgeräts 31 oder zumindest die Entnahme des Deckels 36 z. B. mit Hilfe eines Tasters erkannt wird.

Weiterhin kann das Haushaltsgerät 31 mindestens einen Sensor 43 zur Erfassung von Vitaldaten der das Haushaltsgerät 31 bedienenden Person haben. So kann ein Vitaldatensensor 43 an dem Griff 44 des Gefäßes 32 angeordnet sein, um beispielsweise optisch mit einem Fingerpulssensor die Pulsrate und ggf. die Sauerstoffsättigung des Patienten und/oder mit einer Impedanzmessung beim Ergreifen des Gefäßes 32 an dem Griff 44 andere Vitaldaten, wie beispielsweise den Wassergehalt oder ähnliches zu erfassen.

Die Steuereinheit 41 kann auch beispielsweise durch geeignete Programmierung eingerichtet sein, um in Abhängigkeit von der Steuereinheit 41 zugeführten Vitaldaten die Medikamentengabe anzupassen. Diese Vitaldaten können beispielsweise über die Funkdatenübertragungseinheit 42 von anderen Vitaldatensensoren an das Haushaltsgerät 31 übermittelt werden. Solche Vitaldatensensoren können beispielsweise eine Personenwaage, ein Fitness-Armband des Patienten und dergleichen sein, die drahtlos beispielsweise mit dem Bluetoothoder WLAN-Kommunikationsstandard mit dem Haushaltsgerät 31 kommunizieren.

Bei der Pflege ist die Vitaldatenüberwachung sehr nützlich. Damit kann der persönliche Betreuungsaufwand reduziert und ein eigenständiger Tagesablauf ermöglicht werden.

Es ist daher vorteilhaft, wenn technische Assistenzsysteme in bestehenden Not- und Hilferufsysteme eingebunden werden.

So bedienen sich viele ältere Menschen z. B. deshalb eines Rollators, um das Risiko eines Sturzes zu reduzieren. Hilfreich ist es, wenn auch solche Geräte zur Unterstützung der Fortbewegung, wie ein Rollator, ein Rollstuhl, ein E-Bike oder ähnliches Vitaldatensensoren, eine Datenübertragungseinheit, einen Ortungssensor, eine Lichtsteuerung, eine Ladefunktion, ein Mikrofon und eine Kamera haben. Damit kann mit Hilfe des Vitaldatensensors ein Sturz erkannt und über die Funkdatenübertragungseinheit ein Hilferuf an eine Hausnotrufzentrale abgesetzt werden. Die Not- und Hilferuffunktion kann beispielsweise auch über das Mikrofon durch Sprachkommandos aktiviert werden. Ein Not- bzw. Hilferuf kann auch automatisch ausgelöst werden, wenn bestimmte vorgegebene Indikatormerkmale erkannt wurden. Mit Hilfe der Funkdatenübertragungseinheit und des Mikrofons, eines Lautsprechers und der Kamera kann eine Sprach- und Sichtkommunikation von der Notrufzentrale zur Person hergestellt werden. So kann beispielsweise eine Mobiltelefonfunktion in das Hilfsgerät integriert werden.

Mit den Vitaldatensensoren werden die Vitalwerte überwacht, wie beispielsweise Schwindel oder Müdigkeit. Hierzu können an den Griffen optische Sensoren und/oder Impedanzsensoren eingebaut sein, um Pulsdaten, Sauerstoffsättigungswerte und sonstige Herz- und Kreislaufinformationen des Patienten zu detektieren.

Das Hilfsgerät ist dann eingerichtet, um durch die mechanischen Bewegungen und den Kontakt mit den Griffen durch die Hände des Benutzers das Not- und Hilferufsystem in einen Aktivierungsmodus zu setzen. Wenn beispielsweise ein Rollator in diesem Aktivierungsmodus auf den Boden fällt, auf einen Gegenstand trifft oder in einen übermäßigen Neigungswinkel gerät und dann im Anschluss die Hände den Kontakt zum Rollator verlieren, wird aus diesem "Stand-by-Status" heraus ein Hilferuf- und Notfallmodus aktiviert. In diesem Notfallmodus findet im ersten Schritt eine Herstellung der Kommunikation über einen Funkdatenkanal mit einem in der Nähe befindlichen Mobilfunkgerät oder mit einer entfernten Notrufzentrale statt.

Das Hilfsgerät kann einen Akkumulator zur Versorgung mit elektrischer Energie haben. Die Bewegungsenergie des Hilfsmittels wird beispielsweise mit Hilfe von in die Radnabe integrierten Generatoren genutzt, um die Akkumulatoren während des Gebrauchs wieder aufzuladen. Denkbar ist aber auch, dass andere Elemente zur Gewinnung elektrischer Energie, wie beispielsweise Solarzellen, genutzt werden, um die zum Aufladen der Akkumulatoren benötigte Energie bereitzustellen.

Ein solches Hilfsmittel kann dann mit dem Haushaltsgerät 31 kombiniert werden, um die bedarfsgerechte Ansteuerung des Haushaltgerätes 31 beispielsweise mit Hilfe der Funkdatenübertragungseinheit 42 sicherzustellen.

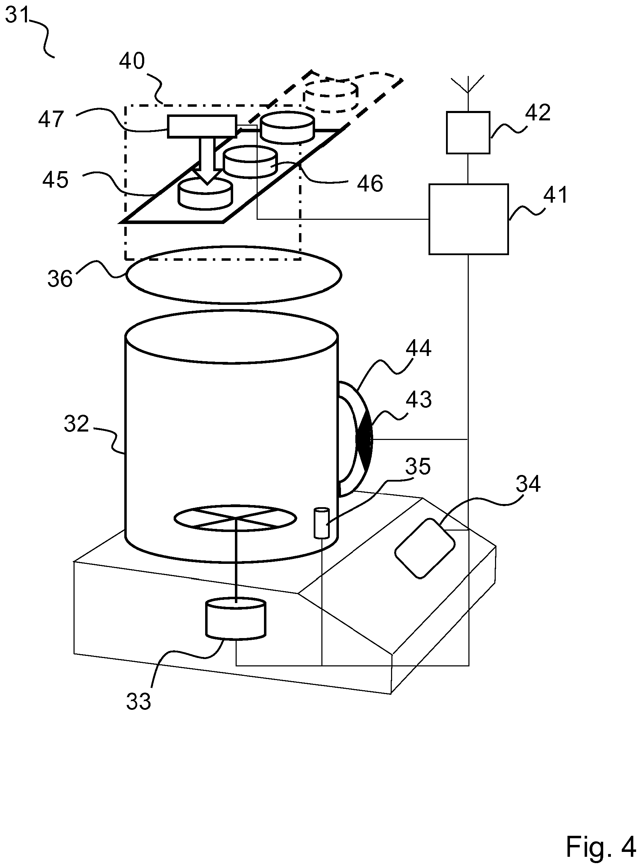

Figur 4 zeigt eine Skizze des Haushaltsgerätes 31 aus Figur 3 mit zur Beimischung von Medikamenten aus Blisterverpackungen 45 eingerichteten Dosiereinheit 40. Die Medikamente werden bereits in Medikamentengaben dosiert in individuell für den Patienten zusammengestellte Verpackungen bereitgestellt. Diese Blisterverpackung 45 wird dann der Dosiereinheit 40 zugeführt, die in Abhängigkeit von der vorgegebenen Einnahmezeit für ein Medikament einen entsprechend hierzu bereitgestellten Blisterabschnitt 46 öffnet und in ein Lebensmittel eindosiert. Die Blisterverpackung 45 (z. B. Blisterstreifen) wird durch die Dosiereinheit 40 mit Förderelementen bewegt, um je nach Tageszeit einen Blisterabschnitt 46 in den Eingriffsbereich einer Blisteröffnungseinheit 47 zu bringen, welche den Blisterabschnitt 46 öffnet, um den flüssigen, zähflüssigen oder festen Inhalt aus dem Blisterabschnitt 46 in das Gefäß 32 zu bringen. Der Blisterabschnitt 46 kann einfach nur mechanisch geöffnet werden, wobei das Medikament aus dem Blisterabschnitt 46 durch Schwerkraft in das Gefäß 32 fällt. Das Medikament kann auch zusätzlich mit einem Stößel aus dem Blisterabschnitt 46 herausbefördert werden. Denkbar ist auch, dass der Blisterabschnitt 46 zur Entnahme des Medikamentes und Einleiten in das Gefäß 32 durchspült wird. Zwischen dem Blisterabschnitt 46 und dem Gefäß 32 kann eine Mahleinheit angeordnet sein, um das Medikament bedarfsweise vor der Eindosierung zu zermahlen.

Anstelle der dargestellten Blisterverpackung 46 in der Art von Blisterstreifen können aber auch einzelne Kapseln bereitgestellt werden. Die Kapseln werden dann tageszeitabhängig von der Dosiereinheit 40 zum Eindosieren der darin enthaltenen Medikamente geöffnet. Diese separaten Kapseln können zur verbesserten Handhabung auch wie ein Blisterstreifen an einem gemeinsamen Träger angeordnet sein. Auch hier sind die Medikamente individuelle und portioniert in eine andere Art von Blisterverpackung 45 in steriler Weise gelagert und können von der Dosiereinheit 40 bedarfsweise gemäß einem für den Patienten individuell vorgegebenen Medikamenten-Zeitplan eindosiert werden.

Die Dosiereinheit 40 kann eine Kühleinheit haben, um die in den Blisterverpackungen 45 bereitgestellten Medikamente bei einer ggf. erforderlichen Temperatur ordnungsgemäß zu lagern.

Die Blisterverpackung 45 muss lediglich in größeren Zeitabschnitten von Pflegepersonal in die Dosiereinheit 40 eingelegt werden, welche diese dann automatisiert und zuverlässig gemäß einem vorgegebenen Medikamentenplan dem Patienten bereitstellt. Eine aufwendige und fehleranfällige manuelle Zusammenstellung und Bereitstellung der Medikamente ist nicht mehr erforderlich. Damit kann der Pflegeaufwand erheblich reduziert und die Pflege ohne Zwischenschaltung von medizinischen Fachpersonal sicherer gestaltet werden.

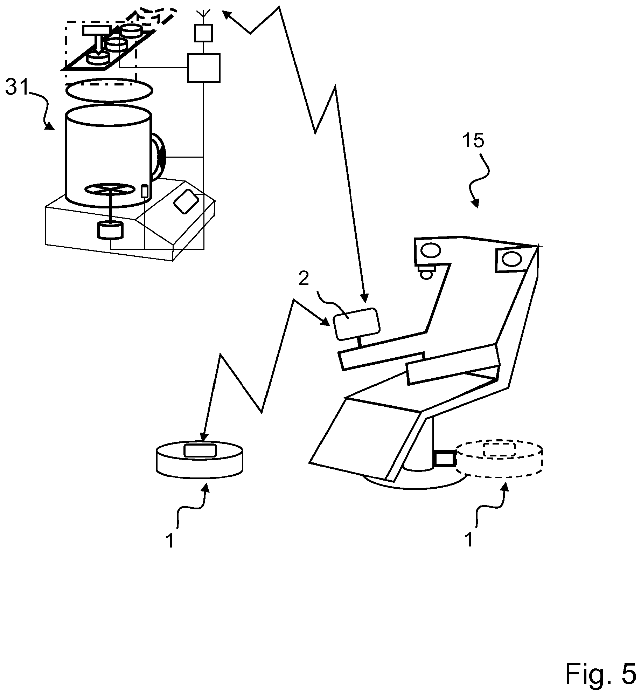

Figur 5 zeigt eine Skizze des Systems aus Sitzmöbel 15, autonomen Haushaltsgerät 1 und Haushaltsgerät 31 zur Lebensmittelversorgung unter Medikamentenbeimischung. Das Sitzmöbel 15 bildet als Lebensmittelpunkt mit seiner Serviceeinheit 2 eine Zentrale, in denen die weiteren Pflegehilfsmittel, wie insbesondere das autonome Haushaltsgerät 1 und das zur Medikamentengabe eingerichtete Haushaltsgerät 31 zusammengeführt und miteinander vernetzt werden. Es bildet hierfür sozusagen eine Datenbrücke und zentrale Logistikeinheit, um beispielsweise als Medikamentenwecker die Medikamenteneinnahme mit Hilfe des Haushaltsgerätes 31 zu steuern und mit Hilfe des mobilen autonomen Haushaltsgerätes 1 die Medikamenteneinnahme zu überwachen. Die Serviceeinheit 2 des Sitzmöbels 15 kann mit dem Haushaltsgerät 31 kommunizieren, um die Medikamentengabe zu steuern oder zumindest Informationen über Medikamentenbeimischung in Lebensmittel abzurufen, um auf dieser Grundlage durch Ansteuerung oder Abfrage des autonomen Haushaltsgerätes 1 und/oder weiterer Vitaldatensensoren die korrekte Medikamenteneinnahme zu überwachen und ggf. bei Abweichungen von vorgegebenen Umgebungs- und/oder Vitaldaten eine Hilfebedürftigkeit zu erkennen. Die Kommunikation der Serviceeinheit 2 des Sitzmöbels 15 mit dem autonomen Haushaltsgerät 1 ermöglicht bedarfsgerechte Überwachungen der Umgebung der Person, um durch die Mobilität des autonomen Haushaltsgerätes 1 dem Tagesablauf der zu betreuenden Person folgen zu können und diese im Hinblick auf eine etwaige Hilfebedürftigkeit unauffällig zu überwachen.

## Patentansprüche

1. Set aus Sitz- oder Liegemöbel (15) für eine zu betreuende Person und einem autonomen Haushaltsgerät, wobei das Sitz- oder Liegemöbel (15) mit einer schwenkbar, verschiebbar und/oder teleskopierbar an dem Sitz- oder Liegemöbel angeordneten Serviceeinheit (2) ausgestattet ist, wobei die Serviceeinheit (2) einen Monitor und eine Eingabeeinheit hat, **dadurch gekennzeichnet, dass** die Serviceeinheit (2) zur Kommunikation mit dem autonomen Haushaltsgerät (1) eingerichtet ist, wobei im Fußbereich des Sitz- oder Liegemöbels eine Ladestation (16) zur Andockung und Aufladung des autonomen Haushaltsgerätes (1) mit Energie angeordnet ist, und wobei das autonome Haushaltsgerät (1) in Form eines Bodenreinigungsroboters oder Rasenmähroboters mit einer Plattform (3) ist, die Fortbewegungsmittel (4), Umgebungssensoren (5a, 5b, 5c) zur Detektion von Eigenschaften der Umgebung des autonomen Haushaltsgerätes (1) und eine Steuerungseinheit (6) hat, wobei die Umgebungssensoren (5a, 5b, 5c) mit der Steuerungseinheit (6) verbunden sind und die Steuerungseinheit (6) zur autonomen Fortbewegung des Haushaltsgerätes (1) durch Ansteuerung der Fortbewegungsmittel (4) in Abhängigkeit von den detektierten Umgebungseigenschaften und zum ständigen Abfahren eines Gebietes zur Ausführung seiner eigentlichen Haushaltstätigkeit eingerichtet ist, wobei
- das autonome Haushaltsgerät (1) eine Kommunikationseinheit (7) zum Aufbau einer Kommunikationsverbindung mit einer entfernt angeordneten Serviceeinheit (2) hat, und
- das autonome Haushaltsgerät (1) ergänzend zur eigentlichen Haushaltstätigkeit zur Erkennung der Hilfebedürftigkeit einer Person in Abhängigkeit der Umgebungseigenschaften eingerichtet ist.

2. Set nach Anspruch 1, **dadurch gekennzeichnet, dass** die Serviceeinheit (2) in eine bewegbare Armlehne (17) oder eine bewegbare Ablage des Sitz- oder Liegemöbels integriert ist.

3. Set nach Anspruch 1 oder 2, **dadurch gekennzeichnet, dass** mindestens ein Mikrofon (19) und/oder Lautsprecher (18) in das Sitz- oder Liegemöbel (15) eingebaut und mit der Serviceeinheit (2) verbunden ist.

4. Set nach einem der Ansprüche 1 bis 3,
**dadurch gekennzeichnet, dass** das autonome Haushaltsgerät (1) mindestens einen Umgebungssensor aufweist, der zur Detektion von Eigenschaften von mit der Bodenreinigungs- oder Rasenmäheinheit aus der Umgebung auffangbaren Elementen ausgebildet ist.

5. Set nach einem der Ansprüche 1 bis 4, **dadurch gekennzeichnet, dass** eine zur Erkennung der Hilfebedürftigkeit einer Person in Abhängigkeit der Umgebungseigenschaften eingerichtete Personenhilfseinheit (8) in das autonome Haushaltsgerät (1) integriert ist, und dass die Personenhilfseinheit (8) zur drahtlosen Übermittlung einer Hilfeanforderung über die Kommunikationseinheit im Falle einer erkannten Hilfebedürftigkeit eingerichtet ist.

6. Set nach einem der Ansprüche 1 bis 4, **dadurch gekennzeichnet, dass** die Serviceeinheit (2) als zur Erkennung der Hilfebedürftigkeit einer Person in Abhängigkeit der über die Kommunikationseinheit empfangbaren Umgebungseigenschaften eingerichtete Personenhilfseinheit (8) ausgebildet ist.

7. Set nach einem der Ansprüche 1 bis 6, **dadurch gekennzeichnet, dass** das autonome Haushaltsgerät (1) mindestens ein Mikrofon (10) zur Erfassung von Schallsignalen hat und die Personenhilfseinheit (8) mittels erfasster Schallsignale zur kommandogesteuerten Fortbewegung zur Schallquelle hin und/oder zur Erkennung der Hilfebedürftigkeit in Abhängigkeit eines Vergleichs erfasster Schallsignale mit vordefinierten Schallmustern eingerichtet ist.

8. Set nach einem der Ansprüche 1 bis 7, wobei das autonome Haushaltsgerät (1) ein Bodenreinigungsroboter ist, **gekennzeichnet durch** einen Auffangbehälter (13) mit einer Grobfiltereinheit (14a), die mit der Bodenreinigungseinheit verbunden und zum Auffangen von auf dem Boden liegenden Medikamenten geeignet ist.

9. Set nach einem der Ansprüche 1 bis 8, **dadurch gekennzeichnet, dass** die Personenhilfseinheit (8) zur Aktivierung einer Hilfeeinheit, die mindestens eine Kamera (5c) und ein Mikrofon (10) umfasst, eingerichtet ist, wenn mittels mindestens eines tastenden Sensors, Gassensors, Lichtsensors und/oder Schallpegelsensors eine Hilfebedürftigkeit erkannt wurde.

10. Set nach einem der Ansprüche 1 bis 9, **dadurch gekennzeichnet, dass** mindestens einer der Umgebungssensoren zur Erkennung von Blut, Urin, Alkohol oder Erbrochenem ausgebildet ist.

11. Set nach einem der Ansprüche 1 bis 10, **dadurch gekennzeichnet, dass** mindestens einer der Umgebungssensoren zur Ermittlung von Raumluftparametern und die Personenhilfseinheit zur Erkennung einer Hilfsbedürftigkeit bei Unterschreiten einer vordefinierten Sauerstoffkonzentration und/oder Erkennung von Uringehalt eingerichtet ist.

12. Set nach einem der Ansprüche 1 bis 11, **dadurch gekennzeichnet, dass** mindestens ein Umgebungssensor in Borsten (12) oder Lappen einer Bodenreinigungseinheit des autonomen Haushaltsgerätes (1) integriert ist.

13. Set nach einem der Ansprüche 1 bis 12, **dadurch gekennzeichnet, dass** das autonome Haushaltsgerät (1) mindestens eine Schnittstelle zur Anbindung von Personen-Vitaldatensensoren hat und zur Auswertung und/oder Übertragung von mit den Personen-Vitaldatensensoren erfassten Sensordaten über die Kommunikationseinheit an eine entfernte Serviceeinheit (2) eingerichtet ist.

14. Set nach einem der Ansprüche 1 bis 13, das weiterhin ein Haushaltsgerät (31) zur Zubereitung von Lebensmitteln mit einem Gefäß (32), insbesondere in Form eines Wasserkochers, eines Garautomaten, eines Teeautomaten, eines Kaffeeautomaten, eines Küchenautomaten oder eines Wassersprudlers, aufweist, wobei die Serviceeinheit (2) des Sitz- oder Liegemöbels (15) zur Kommunikation mit dem Haushaltsgerät (31) ausgebildet ist, **dadurch gekennzeichnet, dass** eine Dosiereinheit (40) mit dem Gefäß (32) verbunden ist, wobei die Dosiereinheit (40) ein Dosiergefäß (37) zur Aufnahme von Medikamenten hat, und wobei eine Steuerungseinheit (41) zur Ansteuerung der Dosiereinheit (40) vorgesehen ist, die zur vorgegebenen Beimischung von Medikamenten aus dem Dosiergefäß (40) zu dem im Gefäß (32) befindlichen Lebensmittel in Abhängigkeit von der Zubereitung eines Lebensmittels eingerichtet ist.

15. Set nach Anspruch 14, **dadurch gekennzeichnet, dass** das Dosiergefäß (37) mehrere voneinander getrennte Medikamentenaufnahmebereiche (38a, 38b) hat, und dass die Dosiereinheit (40) zur gesteuerten Beimischung ausgewählter Medikamente aus den Medikamentenaufnahmebereichen (38a, 38b) eingerichtet ist.

16. Set nach einem der Ansprüche 14 oder 15, **dadurch gekennzeichnet, dass** die Dosiereinheit (40) zur Aufnahme von Blisterverpackungen (45) und zur Eindosierung von in den Blisterverpackungen (45) patientenindividuell und tageszeitabhängig verpackten Medikamenten durch Öffnung der Blisterverpackung (45) und Entnahme und Einleitung der darin enthaltenen Medikamente in das Gefäß (32) ausgebildet ist.

## Claims

1. Set of sitting or reclining furniture (15) for a person to be cared for and an autonomous household appliance (1), wherein the sitting or reclining furniture (15) is equipped with a service unit (2) arranged pivotably, displaceably and/or telescopically on the sitting or reclining furniture, wherein the service unit (2) has a monitor and an input unit, **characterised in that** the service unit (2) is set up for communication with the autonomous household appliance (1), wherein a charging station (16) for docking and charging the autonomous household appliance (1) with energy is arranged in the foot region of the seating or reclining furniture, and wherein the autonomous household appliance (1) is in the form of a floor-cleaning robot or lawn-mowing robot with a platform (3) which has locomotion means (4), environment sensors (5a, 5b, 5c) for detecting properties of the environment of the autonomous household appliance (1) and a control unit (6), wherein the environment sensors (5a, 5b, 5c) are connected to the control unit (6) and the control unit (6) is set up for autonomous locomotion of the household appliance (1) by activating the locomotion means (4) as a function of the detected environmental properties and for continuously travelling through an area in order to carry out its actual domestic activity, wherein
- the autonomous household appliance (1) has a communication unit (7) for establishing a communication link with a remotely arranged service unit (2), and
- the autonomous household appliance (1), in addition to the actual household activity, is set up to recognise the need for assistance of a person depending on the characteristics of the environment.

2. Set according to claim 1, **characterised in that** the service unit (2) is integrated into a movable armrest (17) or a movable shelf of the seating or reclining furniture.

3. Set according to claim 1 or 2, **characterised in that** at least one microphone (19) and/or loudspeaker (18) is built into the sitting or reclining furniture (15) and connected to the service unit (2).

4. Set according to one of claims 1 to 3,
**characterised in that** the autonomous household appliance (1) comprises at least one environmental sensor, which is designed to detect properties of elements that can be collected from the environment by the floor cleaning or lawn mowing unit.

5. Set according to one of claims 1 to 4, **characterised in that** a personal assistance unit (8) set up to detect the need for assistance of a person as a function of the environmental properties is integrated into the autonomous household appliance (1), and **in that** the personal assistance unit (8) is set up for wireless transmission of a request for assistance via the communication unit (7) in the event of a detected need for assistance.

6. Set according to one of claims 1 to 4, **characterised in that** the service unit (2) is designed as a personal assistance unit (8) set up to detect the need for assistance of a person as a function of the environmental properties that can be received via the communication unit (7).

7. Set according to one of claims 1 to 6, **characterised in that** the autonomous household appliance (1) has at least one microphone (10) for detecting sound signals and the personal assistance unit (8) is set up for command-controlled movement towards the sound source by means of detected sound signals and/or for detecting the need for assistance as a function of a comparison of detected sound signals with predefined sound patterns.

8. Set according to one of claims 1 to 7, wherein the autonomous household appliance (1) is a floor cleaning robot, **characterised by** a collecting container (13) with a coarse filter unit (14a), which is connected to the floor cleaning unit and is suitable for collecting medicaments lying on the floor.

9. Set according to one of claims 1 to 8, **characterised in that** the personal assistance unit (8) is set up to activate an assistance unit, which comprises at least one camera (5c) and one microphone (10), when a need for assistance has been detected by means of at least one tactile sensor, gas sensor, light sensor and/or sound level sensor.

10. Set according to one of claims 1 to 9, **characterised in that** at least one of the environmental sensors is designed to detect blood, urine, alcohol or vomit.

11. Set according to one of claims 1 to 10, **characterised in that** at least one of the environmental sensors is designed to determine room air parameters and the personal assistance unit (8) is designed to detect a need for assistance when the oxygen concentration falls below a predefined level and/or to detect urine content.

12. Set according to one of claims 1 to 11, **characterised in that** at least one environmental sensor is integrated in bristles (12) or cloths of a floor cleaning unit of the autonomous household appliance (1).

13. Set according to one of claims 1 to 12, **characterised in that** the autonomous household appliance (1) has at least one interface for connecting personal vital data sensors and is set up for evaluating and/or transmitting sensor data recorded with the personal vital data sensors via the communication unit (7) to a remote service unit (2).

14. Set according to one of claims 1 to 13, which further comprises a household appliance (31) for preparing food with a vessel (32), in particular in the form of a kettle, an automatic cooking machine, an automatic tea machine, an automatic coffee machine, an automatic kitchen machine or a water bubbler, wherein the service unit (2) of the sitting or reclining furniture (15) is designed for communication with the household appliance (31), **characterised in that** the service unit (2) of the sitting or reclining furniture (15) is designed for communication with the household appliance (31), that a dosing unit (40) is connected to the vessel (32), wherein the dosing unit (40) has a dosing vessel (37) for holding medicaments, and wherein a control unit (41) is provided for controlling the dosing unit (40), which is set up for the predetermined admixture of medicaments from the dosing vessel (37) to the foodstuff located in the vessel (32) as a function of the preparation of a foodstuff.

15. Set according to claim 14, **characterised in that** the dosing vessel (37) has a plurality of separate medication receiving areas (38a, 38b), and **in that** the dosing unit (40) is set up for the controlled admixture of selected medications from the medication receiving areas (38a, 38b).

16. Set according to one of claims 14 or 15, **characterised in that** the dosing unit (40) is designed to receive blister packs (45) and to dose medications packaged in the blister packs (45) in a patient-specific and time-of-day-dependent manner by opening the blister pack (45) and removing and introducing the medications contained therein into the vessel (32).

## Revendications

1. Ensemble constitué d'un meuble d'assise ou de couchage (15) pour une personne à assister et d'un appareil ménager autonome, dans lequel le meuble d'assise ou de couchage (15) est équipé d'une unité de service (2) agencée au niveau du meuble d'assise ou de couchage de façon à pouvoir être inclinée, déplacée et/ou télescopée, dans lequel l'unité de service (2) présente un dispositif de surveillance et une unité de saisie, **caractérisé en ce que** l'unité de service (2) est configurée à des fins de communication avec l'appareil ménager autonome (1), dans lequel, dans la zone de pied du meuble d'assise ou de couchage, il est prévu une station de charge (16) pour accueillir et recharger en énergie l'appareil ménager autonome (1), et dans lequel l'appareil ménager autonome (1) est conçu sous la forme d'un robot pour nettoyer les sols ou d'un robot pour tondre le gazon avec une plate-forme (3) qui présente des moyens de déplacement (4), des capteurs d'environnement (5a, 5b, 5c) destinés à détecter des caractéristiques de l'environnement de l'appareil ménager autonome (1), et une unité de commande (6), dans lequel les capteurs d'environnement (5a, 5b, 5c) sont reliés à l'unité de commande (6) et l'unité de commande (6) est configurée pour assurer un déplacement autonome de l'appareil ménager (1) par pilotage des moyens de déplacement (4) en fonction des caractéristiques d'environnement détectées et pour parcourir en permanence une zone permettant d'exécuter son activité ménagère proprement dite, dans lequel
- l'appareil ménager autonome (1) présente une unité de communication (7) destinée à établir une liaison de communication avec une unité de service agencée à distance (2), et
- l'appareil ménager autonome (1) est configuré, en complément de son activité ménagère proprement dite, pour identifier un besoin d'assistance d'une personne en fonction des caractéristiques d'environnement.

2. Ensemble selon la revendication 1, **caractérisé en ce que** l'unité de service (2) est intégrée dans un accoudoir mobile (17) ou dans un élément de support mobile du meuble d'assise ou de couchage.

3. Ensemble selon la revendication 1 ou 2, **caractérisé en ce qu'**un microphone (19) et/ou un haut-parleur (18) au moins est/sont incorporé(s) dans l'unité de service (2) et relié(s) à celle-ci.

4. Ensemble selon l'une des revendications 1 à 3,
**caractérisé en ce que** l'appareil ménager autonome (1) présente au moins un capteur d'environnement qui est configuré pour détecter des caractéristiques d'éléments pouvant être récupéré de l'environnement au moyen de l'unité pour nettoyer les sols ou pour tondre le gazon.

5. Ensemble selon l'une des revendications 1 à 4, **caractérisé en ce qu'**une unité d'assistance à la personne (8) configurée pour identifier un besoin d'assistance d'une personne en fonction des caractéristiques d'environnement est intégrée dans l'appareil ménager autonome (1), et **en ce que** l'unité d'assistance à la personne (8) est configurée pour une transmission sans fil d'une demande d'assistance via l'unité de communication si un besoin d'assistance a été identifié.

6. Ensemble selon l'une des revendications 1 à 4, **caractérisé en ce que** l'unité de service (2) est réalisée comme unité d'assistance à la personne (8) configurée pour identifier le besoin d'assistance d'une personne en fonction des caractéristiques d'environnement pouvant être reçues via l'unité de communication.

7. Ensemble selon l'une des revendications 1 à 6, **caractérisé en ce que** l'appareil ménager autonome (1) présente au moins un microphone (10) pour enregistrer des signaux sonores et **en ce que** l'unité d'assistance à la personne (8), via les signaux sonores détectés, est configurée pour se déplacer par commande d'ordre vers la source sonore et/ou pour identifier le besoin d'assistance en fonction d'une comparaison des signaux sonores enregistrés avec des modèles sonores prédéfinis.

8. Ensemble selon l'une des revendications 1 à 7, dans lequel l'appareil ménager autonome (1) est un robot pour nettoyer les sols, **caractérisé par** un bac collecteur (13) avec une unité de filtrage grossier (14a) qui est reliée à l'unité de nettoyage de sols et qui est adaptée pour collecter des médicaments se trouvant sur le sol.

9. Ensemble selon l'une des revendications 1 à 8, **caractérisé en ce que** l'unité d'assistance à la personne (8) est configurée pour activer une unité d'assistance qui comprend au moins une caméra (5c) et un microphone (10) quand un besoin d'assistance a été identifié à l'aide d'au moins un capteur palpeur, un capteur de gaz, un capteur de lumière et/ou un capteur de niveau sonore.

10. Ensemble selon l'une des revendications 1 à 9, **caractérisé en ce que** l'un au moins des capteurs d'environnement est réalisé pour identifier du sang, de l'urine, de l'alcool ou des vomissures.

11. Ensemble selon l'une des revendications 1 à 10, **caractérisé en ce que** l'un au moins des capteurs d'environnement est configuré pour calculer des paramètres d'air ambiant et **en ce que** l'unité d'assistance à la personne est configurée pour identifier un besoin d'assistance en cas de sous-dépassement d'une concentration d'oxygène prédéfinie, et/ou pour identifier une teneur en urine.

12. Ensemble selon l'une des revendications 1 à 11, **caractérisé en ce que** l'un au moins d'un détecteur d'environnement est intégré dans des brosses (12) ou des chiffons d'une unité de nettoyage de sols de l'appareil ménager autonome (1).

13. Ensemble selon l'une des revendications 1 à 12, **caractérisé en ce que** l'appareil ménager autonome (1) présente au moins une interface destinée à une connexion de capteurs de données vitales de la personne et à une analyse et/ou une transmission, vers une unité de service éloignée (2), de données de capteurs détectées avec les capteurs de données vitales de la personne via l'unité de communication.

14. Ensemble selon l'une des revendications 1 à 13, qui présente en outre un appareil ménager (31) destiné à préparer des aliments avec un récipient (32), en particulier sous la forme d'une bouilloire, d'un cuiseur, d'une machine à thé, d'une machine à café, d'une machine de cuisine ou d'une machine à eau gazeuse, dans lequel l'unité de service (2) du meuble d'assise ou de couchage (15) est réalisée à des fins de communication avec l'appareil ménager (31), **caractérisé en ce qu'**une unité de dosage (40) est reliée au récipient (32), dans lequel l'unité de dosage (40) a un récipient de dosage (37) destiné à recevoir des médicaments, et dans lequel il est prévu une unité de commande (41) destinée à piloter l'unité de dosage (40), qui est configurée pour une incorporation prédéterminée de médicaments provenant du récipient de dosage (40) à l'aliment se trouvant dans le récipient (32) en fonction de la préparation d'un aliment.

15. Ensemble selon la revendication 14, **caractérisé en ce que** le récipient de dosage (37) présente plusieurs zones de réception de médicaments séparées les unes des autres (38a, 38b), et **en ce que** l'unité de dosage (40) est configurée pour une incorporation pilotée de médicaments sélectionnés provenant des zones de réception de médicaments (38a, 38b).

16. Ensemble selon l'une des revendications 14 ou 15, **caractérisé en ce que** l'unité de dosage (40) est réalisée pour recevoir des conditionnements blisters (45) et pour doser des médicaments, conditionnés dans les conditionnements blisters (45) de manière individuelle pour le patient et en fonction des heures de la journée, à travers l'ouverture du conditionnement blister (45), et pour prélever et introduire les médicaments qui y sont contenus jusque dans le récipient (32).
